# FASCICULE DE BREVET EUROPEEN

(11) **EP 1 506 764 B1**
(45) Date de publication et mention de la délivrance du brevet: **11.04.2007**
(21) Numéro de dépôt: 04103849.8
(22) Date de dépôt: 10.08.2004
(51) Int. Cl.: A61K 8/04, A61K 8/19, A61K 8/81, A61K 8/11, A61Q 5/06

(54) **Composition cosmétique comprenant des particules a structure coeur envelope**
Kosmetisches Mittel enthaltend Teichen mit Kern-Schale-Struktur
Cosmetic composition containing particles with core-shell structure

(30) Priorité: 11.08.2003 FR 0350420
(43) Date de publication de la demande: 16.02.2005
(73) Titulaire: L'ORÉAL, 75008 Paris (FR)
(72) Inventeur: Giroud, Franck, 92110, CLICHY (FR)
(74) Mandataire: Poulin, Gérard

(56) Documents cités:
- EP-A- 1 064 918
- WO-A-00/78282
- WO-A-02/16020
- WO-A-02/087525
- WO-A-03/025035
- DE-A- 10 131 173
- FR-A- 2 825 917
- DATABASE CAPLUS [Online] XP002274678 extrait de STN Database accession no. 2002:777045
- DATABASE CAPLUS [Online] XP002274679 extrait de STN Database accession no. 2002:930872
- PATENT ABSTRACTS OF JAPAN vol. 1999, no. 10, 31 août 1999 (1999-08-31) & JP 11 139926 A (POLA CHEM IND INC), 25 mai 1999 (1999-05-25)

## Description

L'invention a trait à une composition cosmétique comprenant des particules à structure coeur-enveloppe (aussi appelées noyau-enveloppe ou "core-shell" en anglais).

L'invention se rapporte également à un procédé cosmétique de traitement des matières kératiniques telles que les cheveux pour leur apporter de la brillance qui met en oeuvre ladite composition.

L'invention a trait en outre à l'utilisation de ladite composition pour apporter de la brillance aux matières kératiniques et en particulier aux cheveux.

Le domaine technique de l'invention peut être défini comme celui des compositions cosmétiques qu'il s'agisse de compositions capillaires ou de compositions pour la peau ou les ongles.

L'utilisation de particules métalliques a déjà été décrite dans différents types de compositions cosmétiques de maquillage.

Ainsi, le document EP-A-1 082 952 décrit-il des compositions de maquillage, notamment des ongles, contenant des particules de verre recouvertes d'une couche métallique permettant l'obtention d'un maquillage présentant un aspect métallique éclatant et résistant à l'usure.

Le document EP-A-953 330, concerne l'association de deux compositions différentes comprenant respectivement des particules métalliques de type pigment goniochromatique et un pigment de type classique ayant une des couleurs du premier pigment pour l'obtention d'un maquillage à effet métallique variable selon l'angle d'observation et présentant des effets irisés.

Plus récemment, la demande de brevet internationale WO-A-02/03913 décrit des compositions de vernis à ongles contenant des particules sous forme de plaquettes d'aluminium dans des proportions pondérales de 0,4 à 0,75% et des agents filmogènes ayant des poids moléculaires élevés pour l'obtention d'un maquillage de type miroir, c'est-à-dire en l'occurrence un maquillage ayant non seulement la couleur de l'aluminium mais également une brillance et une capacité à réfléchir les éléments distincts d'un objet.

Des particules métalliques ont également été incorporées dans des compositions capillaires.

On sait ainsi qu'il est possible d'apporter à la chevelure une brillance supérieure à celle apportée par les corps gras en incorporant dans les compositions capillaires des nanoparticules métalliques, en particulier des nanoparticules d'argent.

De telles compositions sont décrites dans le document EP-A-1 064 918.

Toutefois, il a été constaté que la brillance apportée par de telles compositions s'estompe très rapidement dans le temps.

Dans un autre domaine, le document WO-A-00 78282 décrit l'utilisation de nanoparticules d'argent d'une taille de 1 à 50 nm comme antimicrobien dans des compositions de caoutchouc silicone durcissable.

Ce document ne décrit pas toutefois la mise en oeuvre de nanoparticules encapsulées.

Il existe donc un besoin non encore satisfait pour une composition cosmétique, et en particulier une composition cosmétique capillaire, contenant des particules métalliques qui possède une brillance élevée, cette brillance étant maintenue sur une longue durée et ne s'estompant pas au cours du temps.

Il existe encore un besoin pour une composition cosmétique et en particulier une composition capillaire, qui, tout en présentant une brillance élevée et sur une longue durée possède aussi une grande stabilité dans le temps.

Le but de la présente invention est de fournir une composition cosmétique qui réponde entre autres à ces besoins.

Le but de la présente invention est encore de fournir une composition cosmétique qui ne présente pas les inconvénients, défauts, limitations et désavantages des compositions de l'art antérieur et qui résolve les problèmes des compositions de l'art antérieur.

Ce but, et d'autres encore, sont atteints, conformément à l'invention par une composition cosmétique comprenant, dans un milieu physiologiquement acceptable, au moins un agent présentant une activité cosmétique et des particules comprenant un coeur et une enveloppe solide liée au coeur par une liaison non-covalente, le coeur étant constitué en majorité par au moins un métal, l'enveloppe solide étant constituée par un matériau inorganique, et la taille desdites particules étant inférieure ou égale à 500 nm.

Des compositions cosmétiques, telles que décrites ci-dessus, contenant les particules spécifiques incorporées dans les compositions selon l'invention, qui sont définies par une structure spécifique, des constituants spécifiques et une taille de particule spécifique, n'ont jamais été mentionnées dans l'art antérieur.

De manière étonnante, du fait de l'incorporation dans les compositions de l'invention de ces particules spécifiques, que l'on peut qualifier de nanoparticules métalliques encapsulées, les compositions de l'invention, et en particulier les compositions capillaires, permettent d'obtenir une brillance élevée immédiatement après leur application, c'est-à-dire juste après le traitement du substrat kératinique.

Mais, contrairement aux compositions de l'art antérieur qui contiennent des particules métalliques différentes de celles incorporées dans les compositions de l'invention, c'est-à-dire des particules métalliques non encapsulées, la brillance élevée obtenue avec les compositions de l'invention est conservée pendant une durée prolongée.

A titre d'exemple, cette brillance élevée peut être maintenue pendant une durée pouvant atteindre par exemple un mois ou plus pour des cheveux traités avec les compositions de l'invention alors qu'une composition de l'art antérieur telle que celle décrite dans le document EP-A-1 064 918 incluant des particules non-conformes à l'invention, à savoir non encapsulées, perd la totalité de sa brillance, de son pouvoir réflecteur après une durée de 1 mois.

Outre la conservation de la brillance dans le temps, les compositions cosmétiques selon l'invention présentent une stabilité dans le temps nettement supérieure à celle des compositions de l'art antérieur, représenté par exemple par le document EP-A-1 064 918, qui comprend des particules métalliques différentes de celles incluses dans les compositions de l'invention et, en particulier, non encapsulées.

Il semblerait, mais sans vouloir être lié par aucune théorie, que les particules métalliques spécifiques mises en oeuvre dans les compositions de l'invention limitent l'agrégation des nanoparticules métalliques dans les milieux polaires comme l'eau et/ou l'éthanol et permettent ainsi d'obtenir des dispersions colloïdales d'une grande stabilité, sans séparation de phases.

En outre, la protection apportée par l'enveloppe de matériau inorganique a pour effet d'éviter l'oxydation superficielle du métal ou des métaux constituant en majorité les particules sous l'action des agents extérieurs qu'il s'agisse des agents présents dans la composition, ou des agents avec lesquels les particules sont susceptibles d'être en contact lors de l'application de la composition, comme par exemple le sébum, la transpiration, les larmes, les agents atmosphériques, etc..

En empêchant l'oxydation du métal ou des métaux des particules, on évite la perte de pouvoir réflecteur du métal et la perte de brillance qui sont la conséquence de cette oxydation.

L'invention a trait en particulier à une composition cosmétique capillaire, notamment à une composition cosmétique capillaire pour apporter aux cheveux de la brillance.

L'invention concerne en outre un procédé cosmétique de traitement des matières kératiniques telles que les cheveux en particulier pour apporter de la brillance aux matières kératiniques et en particulier aux cheveux, comprenant l'application sur les matières, fibres kératiniques d'une composition telle que décrite ci-dessus.

L'invention a trait en outre à l'utilisation de la composition telle que décrite ci-dessus pour apporter de la brillance à des matières kératiniques telle que les cheveux.

L'invention concerne enfin l'utilisation des particules spécifiques décrites dans la présente, dans une composition cosmétique, pour apporter de la brillance aux matières, fibres kératiniques telles que les cheveux.

L'invention va maintenant être décrite de manière plus détaillée dans la description qui suit.

Les compositions cosmétiques selon l'invention comprennent au moins un agent présentant une activité cosmétique, ayant un effet cosmétique.

On entend par agent présentant une activité cosmétique, ou actif cosmétique, au sens de la présente invention tout composé actif ayant une activité cosmétique ou dermatologique ou encore tout composé susceptible de modifier l'aspect, le toucher et/ou les propriétés physicochimiques des matières kératiniques telles que les cheveux.

Le ou les agent(s) présentant une activité cosmétique (le (ou les) actif(s) cosmétique(s)) selon l'invention est(sont) généralement choisi(s) parmi :
- les saccharides, les oligosaccharides, les polysaccharides hydrolysés ou non, modifiés ou non,
- les acides aminés, les oligopeptides, les peptides, les protéines hydrolysées ou non, modifiées ou non, les polyacides aminés, les enzymes,
- les acides et alcools gras ramifiés ou non,
- les cires animales, végétales ou minérales,
- les céramides et les pseudo-céramides,
- les acides organiques hydroxylés,
- les filtres UV,
- les antioxydants et les agents anti-radicaux libres,
- les chélatants,
- les agents antipelliculaires,
- les agents régulateurs de séborrée,
- les agents apaisants,
- les tensioactifs cationiques,
- les polymères cationiques, amphotères,
- les silicones organomodifiées ou non,
- les huiles minérales, végétales ou animales,
- les polyisobutènes et poly(α-oléfines),
- les esters,
- les polymères anioniques solubles ou dispersés,
- les polymères non-ioniques solubles ou dispersés,
- les agents réducteurs
- les agents colorants et matières colorantes, et notamment les colorants capillaires,
- les agents moussants,
- les agents filmogènes,
- les particules (autres que les particules à structure coeur-enveloppe de l'invention),
- et leurs mélanges.

Cet agent présentant une activité cosmétique est présent dans une proportion de 0,001 à 10% en poids de la composition cosmétique, de préférence de 0,01 à 5% en poids.

D'une manière générale, les composés de type saccharides, oligosaccharides, polysaccharides hydrolysés ou non, modifiés ou non, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

A titre d'exemples de saccharides, oligosaccharides, polysaccharides hydrolysés ou non, modifiés ou non, utilisables dans l'invention, on peut notamment citer les glucanes, les amidons modifiés ou non (tels que ceux issus, par exemple, de céréales comme le blé, le maïs ou le riz, de légumes comme le pois blond, de tubercules comme les pommes de terre ou le manioc) et différents du bétaïnate d'amidon tel que décrit ci-dessus, l'amylose, l'amylopectine, le glycogène les dextranes, les β-glucanes, les celluloses et leurs dérivés (méthylcelluloses, hydroxyalkylcelluloses, éthylhydroxyéthylcelluloses, carboxyméthyl-celluloses), les fructosanes, l'inuline, la levane, les mannanes, les xylanes, les lignines, les arabanes, les galactanes, les galacturonanes, la chitine, les glucoronoxylanes, les arabinoxylanes, les xyloglucanes, les galactomannanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les gommes de guar et les gommes de xanthane.

Comme acides aminés, on peut citer, par exemple, la cystéine, la lysine, l'alanine, la N-phénylalanine, l'arginine, la glycine, la leucine, et leurs mélanges. A titre d'oligopeptides, de peptides, de protéines hydrolysées ou non, modifiées ou non, que l'on peut utiliser selon l'invention, on peut notamment citer les hydrolysats de protéines de laine ou de soie, modifiées ou non, les protéines végétales telles que les protéines de blé.

Parmi les polyacides aminés utilisables, on peut citer la polylysine.

Parmi les enzymes utilisables, on peut citer les laccases, les peroxydases, les lipases, les protéases, les glycosidases, les dextranases, les uricases, la phosphatase alcaline.

Parmi les acides gras ramifiés ou non convenant dans la présente invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges. Les alcools gras utilisables dans la présente invention, comprennent notamment les alcools en C₈-C₃₀ comme, par exemple, les alcools palmitylique, oléique, linoléique, myristylique, stéarylique et laurylique.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines ou les ozokérites.

Parmi les céramides, on peut citer en particulier les céramides des classes I, II, III et V selon la classification de DAWNING, et plus particulièrement la N-oléyldéshydrosphingosine.

Les acides organiques hydroxylés sont choisis parmi ceux bien connus et utilisés dans la technique. On peut notamment citer l'acide citrique, l'acide lactique, l'acide tartrique, l'acide malique.

Les filtres solaires actifs dans l'UV-A et/ou l'UV-B utilisables selon l'invention sont ceux bien connus de l'homme de métier. On peut notamment citer les dérivés du dibenzoylméthane tels que le 4-méthyldibenzoylméthane, le 4-isopropyldibenzoylméthane, le 4-tert-butyldibenzoylméthane, le 2,4-diméthyldibenzoylméthane, le 4-tert-butyl-4'-diisopropyldibenzoyl-méthane, l'acide p-aminobenzoïque et ses esters tels que le p-diméthylaminobenzoate de 2-éthylhexyle et le p-aminobenzoate d'éthyle N-propoxylé, les salicylates tels que le salicylate de triéthanolamine, les esters d'acide cinnamique tels que le 4-méthoxy-cinnamate de 2-éthylhexyle, le diisopropylcinnamate de méthyle, l'anthranilate de menthyle, les dérivés de benzotriazole, les dérivés de triazine, les dérivés de β,β-diphénylacrylate tels que le 2-cyano-3,3-diphénylacrylate de 2-éthylhexyle et le 2-cyano-3,3-diphénylacrylate d'éthyle, l'acide 2-phénylbenzimidazole-5-sulfonique et ses sels, les dérivés de benzophénone, les dérivés du benzylidènecamphre, les filtres silicones, etc..

A titre d'antioxydants et d'agents anti-radicaux libres utilisables dans la présente invention, on peut citer, par exemple, l'acide ascorbique, des composés ascorbylés tels que le dipalmitate d'ascorbyle, la t-butylhydroquinone, les polyphénols tels que le phloroglucinol, le sulfite de sodium, l'acide érythorbique, les flavonoïdes.

Les chélatants peuvent être choisis notamment parmi l'EDTA (acide éthylènediaminetétraacétique) et ses sels tels que l'EDTA disodique et l'EDTA dipotassique, les composés phosphatés tels que le métaphosphate de sodium, l'hexamétaphosphate de sodium, le pyrophosphate tétrapotassique, les acides phosphoniques et leurs sels tels que les sels de l'acide éthylènediaminetétraméthylènephosphonique.

Les agents antipelliculaires sont choisis, par exemple, parmi :
- le chlorure de benzéthonium, le chlorure de benzalkonium, la chlorhexidine, la chloramine T, la chloramine B, la 1,3-dibromo-5,5-diméthylhydantoïne, la 1,3-dichloro 5,5-diméthylhydantoïne, la 3-bromo 1-chloro 5,5-diméthylhydantoïne, le N-chlorosuccinimide ;
- les dérivés de 1-hydroxy-2-pyridone tels que, par exemple, le 1-hydroxy-4-méthyl-2-pyridone, le 1-hydroxy-6-méthyl-2-pyridone et le 1-hydroxy-4,6-diméthyl-2-pyridone ;
- les trihalogénocarbamides ;
- le triclosan ;
- les composés azolés tels que le climbazole, le kétoconazole, le clotrimazole, l'econazole, l'isoconazole et le miconazole b ;
- les polymères antifongiques tels que l'amphotéricine B ou la nystatine ;
- les sulfures de sélénium ;
- le soufre sous ses différentes formes, le sulfure de cadmium, l'allantoïne, les goudrons de houille ou de bois et leurs dérivés en particulier, l'huile de cade, l'acide undécylénique, l'acide fumarique, les allylamines telles que la terbinafine.

On peut également les utiliser sous forme de leurs sels d'addition d'acides physiologiquement acceptables, notamment sous forme de sels d'acides sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthane sulfonique, propanoïque, 2-oxopropanoïque, propanedioïque, 2-hydroxy-1,4-butanedioïque, 3-phényl-2-propènoïque, α-hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antipelliculaires mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple, l'hydroxyde de triméthylbenzylammonium ; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalino-terreux, comme le magnésium, calcium ; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

Les agents régulateurs de séborrhée sont par exemple le succinylchitosane et la poly-β-alanine.

Les agents apaisants sont par exemple l'azulène et l'acide glycyrrhétinique.

Les tensioactifs cationiques sont ceux bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyl-trialkylammonium, de trialkylbenzylammonium, de trialkylhydroxyalkylammonium ou d'alkylpyridinium ; les dérivés d'imidazoline.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyltriméthylammonium, de méthacrylamidopropyltriméthylammonium ou de diméthyldiallylammonium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique est compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammo-nium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammoniums quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F ;
(13) les polyamines comme le Polyquart® H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous les noms de SALCARE® SC 92, SALCARE® SC 95 et SALCARE® SC 96 par la Société ALLIED COLLOIDS ; et
leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables conformément à l'invention peuvent être choisis parmi les polymères comportant des motifs B et C répartis statistiquement dans 1a chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylates et acrylates, les dialkylaminoalkyl-méthacrylamides et acrylamides. De tels composés sont décrits dans le brevet américain n° 3 836 537. On peut également citer le copolymère acrylate de sodium/chlorure d'acrylamidopropyltriméthylammonium vendu sous la dénomination POLYQUART® KE 3033 par la Société HENKEL.
   Le composé vinylique peut être également un sel de dialkyldiallylammonium tel que le chlorure de diéthyldiallyl-ammonium.
   Les copolymères d'acide acrylique et de ce dernier monomère sont proposés sous les appellations MERQUAT® 280, MERQUAT® 295 et MERQUAT® PLUS 3330 par la société CALGON.
(2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.
   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N-diméthylaminoéthyle, de N-tertio-butylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination AMPHOMER® ou LOVOCRYL® 47 par la société NATIONAL STARCH.
(3) les polyaminoamides réticulés et alkylés partiellement ou totalement dérivant de polyaminoamides de formule générale : 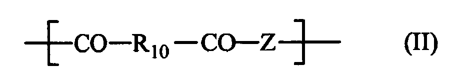 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 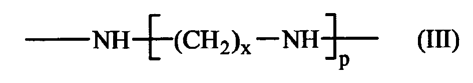 où x=2 et p=2 ou 3, ou bien x=3 et p=2
      ce groupe dérivant de la diéthylènetriamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylènediamine, ou le groupe dérivant de la pipérazine 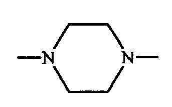
   c) dans les proportions de 0 à 20 % en moles, le groupe -NH-(CH₂)₆-NH- dérivant de l'hexaméthylènediamine, ces polyaminoamides étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 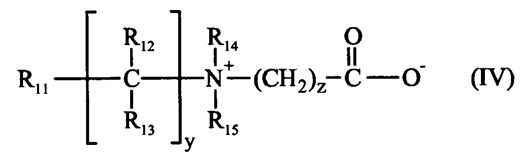 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupement méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de méthacrylate de diméthyl-carboxyméthylammonio-éthyle tel que le produit vendu sous la dénomination DIAFORMER® Z301 par la société SANDOZ.
(5) les polymères dérivés du chitosane comportant des motifs monomères répondant aux formules suivantes: 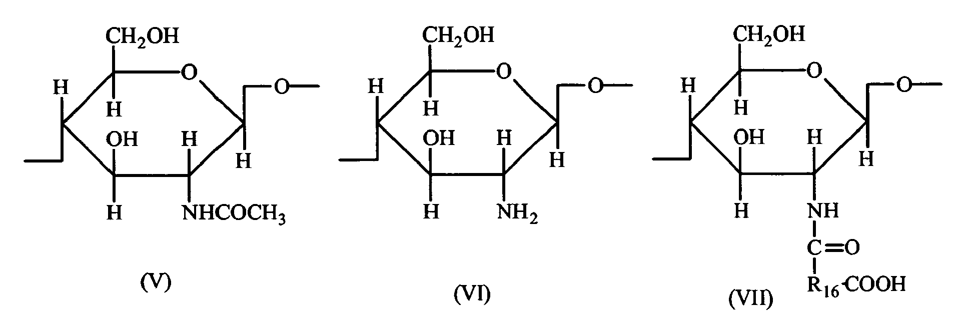 le motif (V) étant présent dans des proportions comprises entre 0 et 30 %, le motif (VI) dans des proportions comprises entre 5 et 50% et le motif (VII) dans des proportions comprises entre 30 et 90%, étant entendu que dans ce motif (VII), R₁₆ représente un groupe de formule: 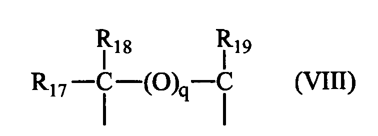 dans laquelle si q=0, R₁₇, R₁₈ et R₁₉, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutylchitosane vendu sous la dénomination EVALSAN® par la société JAN DEKKER.
(7) Les polymères répondant à la formule générale (IX) décrits par exemple, dans le brevet français 1 400 366: 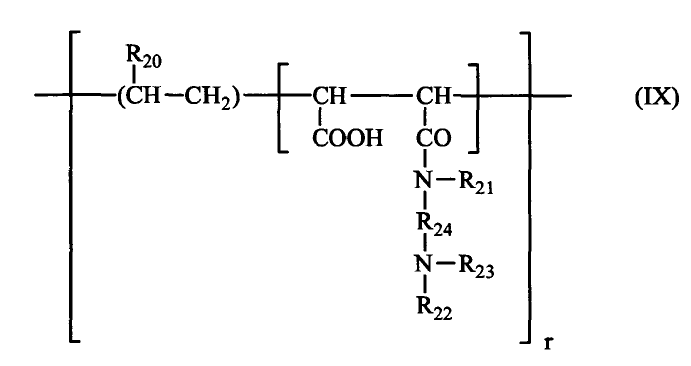 dans laquelle R₂₀ représente un atome d'hydrogène, un groupe CH₃O, CH₃CH₂O, phényle, R₂₁ désigne l'hydrogène ou un groupe alkyle inférieur tel que méthyle, éthyle, R₂₂ désigne l'hydrogène ou un groupe alkyle inférieur tel que méthyle, éthyle, R₂₃ désigne un groupe alkyle inférieur tel que méthyle, éthyle ou un groupe répondant à la formule: -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus, ainsi que les homologues supérieurs de ces groupes et contenant jusqu'à 6 atomes de carbone.
(8) Des polymères amphotères du type -D-X₁-D-X₁- choisis parmi:
   a) les polymères obtenus par action de l'acide chloracétique ou le chloracétate de sodium sur les composés comportant au moins un motif de formule:

      -D-X₁-D-X₁-D- (X)

      où D désigne un groupe 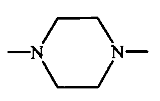 et X₁ désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X₁-D-X₁- (XI)
   où D désigne un groupe 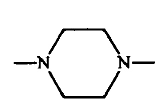 et X₁ désigne le symbole E ou E' et au moins une fois E'; E ayant la signification indiquée ci-dessus et E' étant un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Les silicones utilisables conformément à l'invention peuvent être solubles ou insolubles dans l'eau, et elles peuvent être en particulier des polyorganosiloxanes insolubles dans l'eau ; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 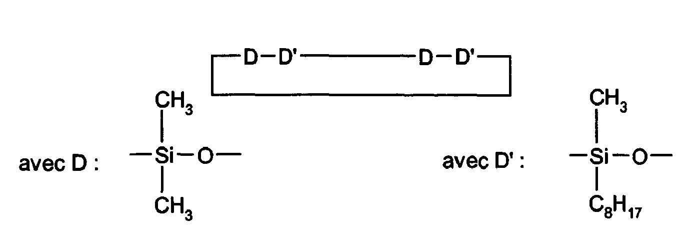
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous les dénominations GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous les dénominations "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorgano-siloxanes décrits dans la demande EP 342 834. On peut citer, par exemple, le produit Q2-8413 de la société DOW CORNING.

On peut notamment citer comme huiles d'origine végétale, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; comme huile d'origine animale, le perhydrosqualène ; comme huiles d'origine minérale, l'huile de paraffine et l'huile de vaseline.

Les polyisobutènes et poly(α-oléfines) sont choisis parmi ceux bien connus dans la technique.

Comme exemples d'esters, on peut notamment citer les esters d'acides gras comme le myristate d'isopropyle, le palmitate d'iso-propyle, le palmitate de 2-éthylhexyle, l'huile de Purcellin (octanoate de stéaryle), l'isononanoate d'isononyle ou d'isostéaryle, le lanolate d'isopropyle, et leurs mélanges.

Les polymères anioniques généralement utilisés dans la présente invention sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 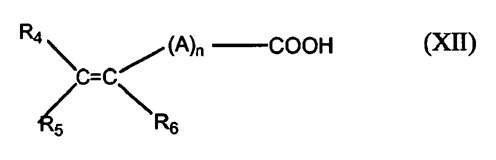 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₄ désigne un atome d'hydrogène, un groupement phényle ou benzyle, R₅ désigne un atome d'hydrogène, un groupement alkyle inférieur ou carboxyle, R₆ désigne un atome d'hydrogène, un groupement alkyle inférieur, un groupement -CH₂-COOH, phényle ou benzyle.

Dans la formule (XII) ci-dessus, un groupement alkyle inférieur comporte de préférence de 1 à 4 atomes de carbone et désigne en particulier, les groupements méthyle et éthyle.

Les polymères anioniques à groupements carboxyliques préférés selon l'invention sont:
A) les homo- ou copolymères d'acide acrylique ou méthacrylique ou leurs sels, et en particulier les produits vendus sous les dénominations VERSICOL® E ou K par la société ALLIED COLLOID, ULTRAHOLD® par la société BASF Les copolymères d'acide acrylique et d'acrylamide vendus sous la forme de leur sel de sodium sous les dénominations RETEN® 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER® par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER® MAEX par la société BASF.
C) les copolymères dérivés d'acide crotonique tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000 vendus respectivement sous les dénominations Flexan® 500 et Flexan® 130 par National Starch. Ces composés sont décrits dans le brevet FR 2198719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER® HSP 1180 par Henkel.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique monoestérifié vendu sous la dénomination GANTREZ® ES 425 par la société ISP, les copolymères d'acide méthacrylique et de méthacrylate de méthyle vendus sous la dénomination EUDRAGIT® L par la société ROHM PHARMA, le copolymère d'acide méthacrylique et d'acrylate d'éthyle vendu sous la dénomination LUVIMER® MAEX par la société BASF, le copolymère acétate de vinyle/acide crotonique vendu sous la dénomination LUVISET® CA 66 par la société BASF et le terpolymère acétate de vinyle/acide crotonique/polyéthylèneglycol vendu sous la dénomination ARISTOFLEX® A par la société BASF.

Selon l'invention, on peut également utiliser les polymères anioniques sous forme de latex ou de pseudolatex, c'est-à-dire sous forme d'une dispersion aqueuse de particules de polymères insolubles.

À titre de polymères non ioniques utilisables selon la présente invention, on peut notamment citer :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX® 50 000, PEOX® 200 000 et PEOX® 500 000 ;
- les homopolymères d'acétate de vinyle tels que le produit proposé sous le nom de APPRETAN® EM par la société HOECHST ou le produit proposé sous le nom de RHODOPAS® A 012 par la société RHONE POULENC ;
- les copolymères d'acétate de vinyle et d'ester acrylique tels que le produit proposé sous le nom de RHODOPAS® AD 310 de RHONE POULENC ;
- les copolymères d'acétate de vinyle et d'éthylène comme le produit proposé sous le nom de APPRETAN® TV par la société HOECHST;
- les copolymères d'acétate de vinyle et d'ester maléique, par exemple de maléate de dibutyle tels que le produit proposé sous le nom de APPRETAN® MB EXTRA par la société HOECHST ;
- les copolymères de polyéthylène et d'anhydride maléique ;
- les homopolymères d'acrylates d'alkyle et les homopolymères de méthacrylates d'alkyle tels que le produit proposé sous la dénomination MICROPEARL® RQ 750 par la société MATSUMOTO ou le produit proposé sous la dénomination LUHYDRAN® A 848 S par la société BASF ;
- les copolymères d'esters acryliques tels que, par exemple, les copolymères d'acrylates d'alkyle et de méthacrylates d'alkyle comme les produits proposés par la société ROHM & HAAS sous les dénominations PRIMAL® AC-261 K et EUDRAGIT® NE 30 D, par la société BASF sous les dénominations ACRONAL® 601, LUHYDRAN® LR 8833 ou 8845, par la société HOECHST sous les dénominations APPRETAN® N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL® LX 531 8 par la société NIPPON ZEON ou ceux proposés sous la dénomination CJ 0601 8 par la société ROHM & HAAS ;
- les polyuréthannes tels que les produits proposés sous les dénominations ACRYSOL® RM 1020 ou ACRYSOL® RM 2020 par la société ROHM & HAAS, les produits URAFLEX® XP 401 UZ, URAFLEX® XP 402 UZ proposés par la société DSM RESINS ;
- les copolymères d'acétate d'alkyle et d'uréthanne tels que le produit 8538-33 par la société NATIONAL STARCH ;
- les polyamides tels que le produit ESTAPOR® LO 11 proposé par la société RHONE POULENC ;
- les gommes de guar non ioniques chimiquement modifiées ou non modifiées.

Les gommes de guar non ioniques non modifiées sont, par exemple, les produits vendus sous la dénomination VIDOGUM® GH 175 par la société UNIPECTINE et sous la dénomination JAGUAR® C par la société MEYHALL.

Les gommes de guar non-ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont, par exemple, vendues sous les dénominations commerciales JAGUAR® HP8, JAGUAR® HP60 et JAGUAR® HP120, JAGUAR® DC 293 et JAGUAR® HP 105 par la société MEYHALL, ou sous la dénomination GALACTASOL® 4H4FD2 par la société AQUALON.

Les groupes alkyle des polymères non ioniques comportent de préférence de 1 à 6 atomes de carbone.

Les agents réducteurs peuvent être choisis parmi les thioacides et leurs sels (acide thioglycolique ou thiosulfate, cystéine ou cystéamine), les sulfites de métaux alcalins ou alcalinoterreux, les sucres réducteurs tels que le glucose, la vitamine C et ses dérivés, les dérivés d'acide sulfovinique, les phosphines.

Les agents colorants peuvent être choisis parmi les structures conjuguées linéaires ou aromatiques (hétérocycliques ou non). Parmi celles-ci, on peut citer les colorants benzéniques nitrés, les colorants aromatiques, les colorants amino-benzéniques, les colorants azoïques, les colorants anthraquinoniques, les diamines aromatiques, les aminophénols, les phénols et naphtols, les porphyrines, tétraphénylporphyrines, métalloporphyrines, les phtalocyanines, les caroténoïdes, les flavonoïdes, les molécules fluorescentes (fluoresceine, rhodamine, coumarine ...).

Les agents filmogènes peuvent être choisis parmi les polymères filmogènes, par exemple ceux décrits dans FR-2 739 022, FR-2 757 048 ou FR-2 767 699 appartenant à l'Oréal.

Les agents moussants peuvent être choisis généralement parmi les tensioactifs au caractère moussant, les polymères cationiques et les polymères anioniques aux propriétés moussantes ; ou encore, il peut s'agir d'un agent spécifique tel que celui décrit dans FR-2 751 221 appartenant à l'Oréal.

Les particules, en tant qu'actifs cosmétiques, sont autres que les particules à structure coeur-enveloppe de l'invention et peuvent être choisis parmi les particules organiques, minérales ou en composite.

Les compositions cosmétiques selon l'invention sont essentiellement caractérisées par les particules qu'elles contiennent. Ces particules sont selon l'invention des particules qui peuvent être définies comme étant des nanoparticules métalliques encapsulées par un matériau inorganique.

Au sens de la présente invention, on entend par "nanoparticules" des particules dont la taille est inférieure ou égale à 500 nm, de préférence comprise entre 1 nm et 500 nm, de préférence encore comprise entre 1 nm et 100 nm, mieux de 1 nm à 50 nm.

On entend par "taille de particule" la dimension maximale qu'il est possible de mesurer entre deux points de la particule. De telles tailles sont mesurables directement par des techniques de microscopie comme la microscopie à balayage électronique ou la microscopie à force atomique, ou par des techniques indirectes comme la diffusion dynamique de la lumière.

Les particules incorporées dans les compositions de l'invention peuvent avoir des formes variées ; elles peuvent revêtir par exemple la forme de sphères, de lamelles, de fibres, de tubes, de polyèdres, elles peuvent aussi avoir une forme totalement aléatoire. Une forme préférée est sphérique.

Les particules incorporées dans les compositions de l'invention ont un coeur, noyau ("core") constitué en majorité par au moins un métal.

Par métal, on entend un corps simple constitué uniquement d'atomes d'un élément métallique susceptible d'engendrer des cations.

Par "en majorité", on entend que le coeur de la particule est constitué par 50% ou plus en masse d'au moins un métal.

De préférence, le coeur est constitué par au moins 80% en masse, de préférence encore par au moins 90%, en masse et mieux par 100% en masse d'au moins un métal.

On entend généralement par métal, l'aluminium et tous les éléments de numéro atomique allant de 21 à 82 et composant les colonnes 3 à 13 de la classification périodique des éléments selon la nouvelle notation IUPAC : on pourra à ce sujet se référer au "CRC Handbook of Chemistry and Physics", 80th Print Edition.

Le terme métal inclut également tous les alliages de ces éléments, et les mélanges de ces métaux et alliages.

Le coeur peut, ainsi également, être constitué dans les pourcentages cités ci-dessus par un mélange de deux ou plus de ces métaux et/ou alliages de ceux-ci.

Le coeur ou noyau peut aussi être un coeur composite constitué de plusieurs zones, des zones adjacentes étant constituées par des métaux, alliages ou mélanges différents.

Des coeurs composites préférés sont des coeurs multicouches comprenant un coeur ou noyau interne formant substrat constitué par un métal, alliage ou mélange de métaux ou d'alliages recouvert au moins partiellement par une première couche en un métal, alliage de métal ou mélange de métaux ou d'alliages différent de celui constituant le coeur ou noyau interne, et éventuellement par une ou plusieurs autres couches, chacune de ces couches recouvrant au moins partiellement la couche précédente et chaque couche étant constituée par un métal, alliage ou mélange de métaux ou d'alliages différent de la couche suivante (si celle-ci existe) et de la couche précédente.

Outre ledit métal ou lesdits métaux, le coeur ou noyau peut comprendre en outre des impuretés inévitables, et des stabilisants de toute sorte.

Le noyau ou coeur peut aussi comprendre par exemple en outre des composés métalliques autres que des métaux tels que des oxydes de métaux.

Ainsi, dans le cas de l'aluminium, le noyau ou coeur peut comprendre de l'alumine Al₂O₃ dans une proportion par exemple de 10%, pour 90% de Al métal.

Le métal est choisi de préférence parmi les métaux de transitions, les métaux des terres rares et leurs alliages et mélanges.

De préférence encore, le métal est choisi parmi l'aluminium, le cuivre, l'argent, l'or, l'indium, le fer, le platine, le nickel, le molybdène, le titane, le tungstène, l'antimoine, le palladium, le zinc, l'étain et leurs alliages et mélanges.

Parmi la liste précédente, les métaux dits "nobles" et le cuivre sont préférés. On entend par métaux nobles, l'or, l'argent, le palladium, le platine et leurs alliages et mélanges.

L'argent est le métal préféré entre tous.

L'enveloppe peut être en contact direct avec le métal natif qui constitue en majorité le coeur ou le noyau, en d'autres termes, aucune sous-couche n'est alors interposée entre l'enveloppe et le métal ; ou bien, le coeur ou noyau constitué en majorité par au moins un métal natif peut, avant son encapsulation, avant la formation de l'enveloppe, être modifié en surface par un traitement modifiant les propriétés de celle-ci. Ce traitement peut consister à stabiliser la surface du noyau ou coeur (c'est-à-dire la surface du métal) par une monocouche adsorbée ou liée par covalence.

Selon l'invention, l'enveloppe entourant le coeur (pourvu ou non d'une couche telle que décrite ci-dessus) est constituée par un matériau inorganique.

Selon l'invention, ce matériau est un matériau solide à température ambiante.

Il n'existe aucune limitation quant à la nature du matériau inorganique.

De préférence, le matériau inorganique est choisi parmi les matériaux constitués par les oxydes métalliques et les polymères organométalliques.

Les oxydes métalliques sont choisis de préférence parmi les oxydes de silicium, de titane, de cérium, d'aluminium, de zirconium, de zinc, de bore, de lithium, de magnésium, de sodium, les oxydes mixtes de ceux-ci, et les mélanges de ces oxydes et oxydes mixtes.

Les oxydes métalliques plus particulièrement préférés sont la silice, l'oxyde de titane et l'alumine.

Les polymères organométalliques sont choisis, de préférence, parmi les produits issus de la polycondensation d'alcoxysilanes.

L'enveloppe, la capsule constituée par un matériau inorganique a généralement une épaisseur de 2 à 300 nm, de préférence de 5 à 250 nm, de préférence encore de 10 à 100 nm.

Il est à noter que cette enveloppe, cette capsule, et conformément à la définition bien connue de l'encapsulation dans le domaine de la technique, n'est pas une monocouche, une couche moléculaire mais bien une couche que l'on peut qualifier de paroi "épaisse" dont l'épaisseur est généralement comprise dans la plage définie ci-dessus.

Selon l'invention, la capsule, enrobage ou enveloppe, est reliée au noyau ou coeur par une liaison physique, sans liaisons covalentes. En d'autres termes, dans les particules incorporées dans les compositions de l'invention, l'interface noyau/enveloppe est définie comme ne présentant pas de liaisons covalentes.

La formation de l'enveloppe, de la capsule, autour du noyau métallique, dans les particules des compositions de l'invention peut être réalisée par divers procédés.

Ces procédés qui sont généralement désignés par les termes de procédé d'encapsulation ou de nanoencapsulation sont connus de l'homme du métier dans ce domaine de la technique et peuvent être généralement divisés en deux grandes familles : à savoir d'une part les procédés physico-chimiques et d'autre part les procédés chimiques.

Les procédés physico-chimiques peuvent être choisis parmi la séparation de phase ou coacervation, la précipitation contrôlée, et tous les autres procédés physico-chimiques de microencapsulation connus.

Les procédés chimiques peuvent être choisis parmi la polycondensation interfaciale, la polycondensation in-situ, la polymérisation en émulsion, et tous les autres procédés chimiques de microencapsulation connus.

Pour plus de détails sur ces procédés d'encapsulation, on pourra se reporter au document "Microencapsulation Methods and Industrial Applications", (ISBN 0-8247-9703-S).

Parmi les divers procédés d'encapsulation exploitables est préférée l'encapsulation par un procédé sol-gel.

De ce fait, le matériau inorganique qui constitue l'enveloppe des particules est de préférence choisi parmi les matériaux inorganiques susceptibles d'être obtenus par un procédé sol-gel, de préférence encore parmi les oxydes métalliques et les polymères organométalliques susceptibles d'être obtenus par un procédé sol-gel à partir d'un ou de plusieurs précurseurs.

En particulier, le matériau inorganique qui constitue l'enveloppe des particules est choisi parmi les oxydes métalliques et les polymères organométalliques susceptibles d'être obtenus, synthétisés par polycondensation d'un ou de plusieurs précurseurs alcoxydes métalliques choisis, de préférence parmi les alcoxydes de silicium, d'aluminium, de bore, de lithium, de magnésium, de titane, de zirconium, et les alcoxydes mixtes de ceux-ci.

Pour plus de détails sur la nature des précurseurs et les mécanismes réactionnels, on pourra se référer à l'ouvrage « Sol Gel Science » publié par C. J. BRINKER et G. W. SCHERER aux éditions Academic Press (ISBN 0-12-134970-5).

Un tel procédé sol-gel permet d'obtenir un système noyau(coeur)-enveloppe (« core/shell ») constitué par un noyau métallique et une capsule d'oxyde métallique ou de polymère organométallique d'une épaisseur généralement supérieure à 2 nm et classiquement comprise entre 2 nm et 300 nm. Pour plus de détails sur le procédé d'encapsulation sol-gel, on pourra se référer aux articles suivants :
« Synthesis and Self Assembly of Au/SiO₂ Core-Shell Colloids" (Nano Letters 2002, 2 (7), 785-788).
"Cocondensation of Organosilica Hybrid Shells on Nanoparticle Templates : A direct Synthetic Route to Functionalized Core-Shell Colloids" (Langmuir 2000, 16, 1454-1456).
"Synthesis and Characterization of Gold-Silica Nanoparticles Incorporating a Mercaptosilane Core-Shell Interface" (Langmuir 2002, 18, 8566-8572).

Ces précurseurs sont de préférence des alcoxysilanes.

On entend par alcoxysilane des molécules comprenant un, deux ou trois atomes de silicium et au moins deux fonctions hydroxy ou hydrolysables, telles que méthoxy, éthoxy, propoxy, etc. L'alcoxysilane peut éventuellement comprendre en outre des fonctions lui assurant une compatibilité avec le milieu physiologiquement acceptable, en particulier avec solvant de celui-ci, et/ou une affinité avec les matières ou fibres kératiniques. Parmi ces fonctions, on peut citer les principales fonctions améliorant la solubilité dans l'eau telles que les fonctions alkyl amine, alkyl alcool, alkyl thiol, alkyl acide, alkyl polyamine, alkyl polyol et alkyl polycarboxylique. Parmi les alcoxysilane hydrosoluble utilisables comme précurseur de la capsule, on peut citer de façon non exhaustive le 3-aminopropyltriéthoxysilane, le 3-aminopropyl-méthyl-diéthoxysilane, le 3-[bis(hydroxyéthyl)aminopropyl]triéthoxysilane, etc.

Parmi ces précurseurs, de l'enveloppe, de la capsule, le tétraéthylorthosilicate (TEOS) est préféré.

Dans une forme de réalisation de l'invention, les particules enrobées, encapsulées incluses dans les compositions de l'invention sont susceptibles d'être préparées par un procédé dans lequel on réalise, dans un milieu aqueux, la condensation sur des particules constituées en majorité par au moins un métal, lesdites particules étant destinées à former le coeur des particules « enrobées » coeur-enveloppe que l'on souhaite obtenir, de composés organiques du silicium soluble dans l'eau, peu ou pas polymérisés, choisis parmi les organosilanes comportant un atome de silicium et les organosiloxanes comportant deux ou trois atomes de silicium, ces composés organiques du silicium comportant en outre dans ce mode de réalisation au moins une fonction chimique basique et au moins deux groupes hydrolysables ou hydroxyles par molécule, ces composés organiques du silicium, peu ou pas polymérisés, étant neutralisés à raison de 1/1000 à 99/100, de préférence de 0,2/100 à 70/100 par un agent de neutralisation. Ce procédé est décrit dans le document FR-A-2 783 164 a la description duquel on pourra se référer.

Dans une autre forme de réalisation de l'invention, les particules enrobées, encapsulées incluses dans les compositions de l'invention sont susceptibles d'être préparées par un procédé dans lequel on réalise, dans un milieu aqueux, la condensation sur des particules constituées en majorité par au moins un métal, lesdites particules étant destinées à former le coeur de la particule « enrobée » coeur-enveloppe que l'on souhaite obtenir, de composés organiques du silicium solubles dans l'eau, peu ou pas polymérisés, choisis dans cette forme de réalisation parmi les organosilanes comportant un atome de silicium et les organosiloxanes comportant deux ou trois atomes de silicium, ces composés organiques du silicium comportant par molécule en outre au moins deux groupes hydroxyles ou deux groupes fonctionnels hydrolysables et au moins deux groupes fonctionnels non-hydrolysables, un au moins de ces groupes fonctionnels non-hydrolysables étant un groupe fonctionnel ayant un effet cosmétique et au moins un autre de ces groupes fonctionnels non-hydrolysables étant un groupe fonctionnel solubilisant.

Ce procédé est décrit dans le document FR-A-2 783 165 à la description duquel on pourra se référer.

De préférence, le ou les groupe(s) à effet cosmétique est(sont) un(des) groupe(s) à fonction colorante, de protection anti-UV, anti-bactérienne ou anti-fongique ou à fonction réductrice.

Dans encore une autre forme de réalisation de l'invention, les particules enrobées, encapsulées incluses dans les compositions de l'invention sont susceptibles d'être préparées par un procédé dans lequel on réalise, dans un milieu aqueux, la condensation sur des particules constituées en majorité par au moins un métal natif, lesdites particules étant destinées à former le coeur de la particule « enrobée » coeur-enveloppe que l'on souhaite obtenir, de composés organiques du silicium solubles dans l'eau, peu ou pas polymérisés, choisis parmi les organosilanes comportant un atome de silicium et les organosiloxanes comportant deux ou trois atomes de silicium, ces composés organiques du silicium dans ce mode de réalisation comportant en outre au moins une fonction chimique solubilisante non basique et au moins deux groupes hydrolysables par molécule.

Ce procédé est décrit dans le document WO-A-01/22931 à la description duquel on pourra se référer.

Dans une autre forme de réalisation, les particules enrobées, encapsulées, incluses dans les compositions de l'invention sont susceptibles d'être préparées par un procédé comprenant les étapes suivantes :
a) on prépare un mélange
   (i) d'une dispersion de particules constituées en majorité par au moins un métal natif et destinées à former le coeur de la particule « coeur-enveloppe » que l'on souhaite obtenir, dans un milieu liquide, notamment un milieu aqueux ou alcoolique ou huileux, éventuellement en présence d'un dispersant,
   (ii) et d'une solution d'un matériau hybride organo-minéral réticulé, ledit matériau (avant hydrolyse) étant obtenu par voie sol-gel à partir d'un prémélange comprenant:
      (A) au moins un composé métallique ou métallo-organique, et
      (B) au moins un polymère organique fonctionnalisé ou un précurseur de celui-ci, ou au moins un polymère siliconé fonctionnalisé ou un précurseur de celui-ci, ce dernier étant différent de (A),
b) ledit mélange est mis en contact avec de l'eau lorsque ladite dispersion des particules destinées à former le coeur est une dispersion dans un milieu liquide alcoolique ou huileux.

Le matériau hybride organe-minéral, utilisé pour le procédé permettant d'obtenir l'enveloppe solide des particules de la composition de l'invention dans ce mode de réalisation, est connu et a été décrit en particulier dans la demande de brevet français n° 97 04157 et dans la demande de brevet français FR-A-2 825 917 à la description de laquelle on pourra se référer.

La surface externe des particules, c'est-à-dire la surface externe de la capsule, de l'enveloppe, peut être modifiée de façon covalente par au moins un groupement chimique qui est susceptible d'améliorer l'adsorption des particules sur les matières kératiniques telles que les cheveux. La surface externe des particules, c'est-à-dire la surface externe de la capsule, de l'enveloppe des particules peut être modifiée aussi de façon covalente par au moins un groupement chimique capable de réagir chimiquement avec les matières kératiniques telles que les cheveux.

Dans le premier cas, l'adsorption sur les matières kératiniques telles que les cheveux des nanoparticules "core shell" des compositions de l'invention peut être améliorée en modifiant de façon covalente la capsule de matériau organique tel qu'un polymère par différents groupements chimiques (groupe A ci-dessous) rendant la surface des particules par exemple, plus hydrophobe, et/ou plus cationique, et/ou plus anionique, et/ou plus hydrophile.

On définit l'adsorption comme mettant en oeuvre des énergies de liaisons plus faibles que des liaisons covalentes, c'est-à-dire inférieure à 50 kcal/mol entre la matière kératinique telle que le cheveu et la particule. Ces liaisons de faibles énergies sont par exemple les forces de Van der Waals, liaisons hydrogènes, complexes donneur-accepteur d'électrons, etc...

Le groupement capable d'améliorer l'adsorption des particules sur les matières kératiniques est généralement choisi parmi les groupements du groupe A suivant :

### Groupe A :

- Acides carboxyliques et leurs sels,
- Amines primaires, secondaires, tertiaires et quaternaires,
- Phosphates,
- Oxydes de soufre tels que sulfones, sulfoniques, sulfoxides, et sulfates,
- Noyaux aromatiques tels que phényle, triazine, thiophène et imidazole.

Dans le second cas, on peut également favoriser l'adhésion sur les matières kératiniques telles que les cheveux des nanoparticules de l'invention en modifiant de façon covalente la capsule de matériau inorganique, par différents groupements (groupe B) capables de réagir chimiquement sur la matière kératinique. Plus spécifiquement, on entend par groupements possédant une réactivité sur la matière kératinique en particulier le cheveu, les groupements capables de former une liaison covalente avec cette matière par exemple avec les amines et/ou les acides carboxyliques et/ou les thiols des acides aminés constituant la matière kératinique. La formation de ces liaisons covalentes peut être soit spontanée, soit se produire par une activation par la température, le pH, la lumière, un co-réactif, ou par un catalyseur chimique ou biochimique tel qu'un enzyme.

Le groupement susceptible, capable de réagir chimiquement avec les matières kératiniques telles que les cheveux est généralement choisi parmi les groupements du groupe B suivant :

### Groupe B :

- Epoxydes,
- Vinyle et vinyle activé : acrylonitrile, esters acrylique et méthacrylique, acide et esters crotonique, acide et esters cinnamique, styrène et dérivés, butadiène, éthers de vinyle, vinyle cétone, esters maléique, maléimides, vinyl sulfones, ...
- Acides carboxyliques et leurs dérivés : anhydride, chlorure d'acide, esters,
- Acétals, hémi-acétals,
- Aminals, hémi-aminals,
- Cétones et alpha-hydroxycétones, alpha-halocétones,
- Lactones, thiolactones,
- Isocyanates,
- Thiocyanates,
- Imines,
- Imides (succinimides, glutimides),
- Pyridyldithio,
- N hydroxysuccinimide esters,
- Imidates,
- Oxazine et oxazoline,
- Oxazinium et oxazolinium,
- Groupements de formule R₁X, dans laquelle R₁ est un groupe alkyle de 1 à 30C, un groupe aryle comprenant de 6 à 30C, un groupe aralkyle de 7 à 30C (groupe alkyle de 1 à 30C), et X est un groupe partant tel que I, Br, Cl, OSO₃R où R est H, un groupe alkyle de 1 à 30C, -SO₂R' où R' est H, un groupe alkyle de 1 à 30C, un groupe tosyle, N(R'')₃ où R'' est un groupe alkyle de 1 à 30C, OPO₃(R''')₂ où R''' est H ou un groupe alkyle de 1 à 30C ; des groupements de formule R₁X préférés sont les halogénures d'alkyle, d'aryle ou d'aralkyle ;
- Groupements de formule R₂X où R₂ représente un cycle carboné de 3 à 30C ou un hétérocycle insaturé de 3 à 20 chaînons comprenant un ou plusieurs hétéroatomes choisis parmi N, S, O et P, et X est un groupe partant tel que défini ci-dessus ; des groupements de formule R₂X préférés sont les halogénures de cycles insaturés tels que la chlorotriazine, la chloropyrimidine, la chloroquinoxaline, et le chlorobenzotriazole,
- Groupements de formule : R₃SO₂X où R₃ a la même signification que R₁ et X représente un groupe partant et a la signification déjà donnée ci-dessus,
- Lactones,
- Thiolactones,
- Siloxanes.

A titre d'exemple, de façon non exhaustive, on peut citer l'activation par le N-hydroxysulfosuccinimide de particles coeur/enveloppe ("core/shell") possédant un coeur en argent et une capsule en oxyde de titane obtenue par polycondensation de tétraéthoxytitanate. Les groupements sulfosuccinimides greffés à la surface des nanoparticules des compositions de l'invention permettent de lier de façon covalente les nanoparticules des compositions de l'invention aux cheveux par reaction sur les amines libres superficielles que possède la fibre capillaire.

Pour plus de détails sur ce procédé d'activation, on pourra se référer au document suivant : « Biofunctionalised Biocompatible Titania Coatings for Implants ». Key Eng. Mat. 206-213, (2002), 1547-1550.

A noter que les fonctions chimiques sur la surface de la matière kératinique par exemple de la fibre capillaire peuvent être densifiées par un pré-traitement de la fibre par une solution de polymère ayant une affinité particulière pour la fibre et présentant des fonctions réactives. Dans le précédent exemple, la densité des fonctions amines à la surface de la fibre peut être augmentée, par exemple, en absorbant préalablement de la polyéthylèneimine.

Afin d'accroître la durabilité de l'effet dans le temps, outre l'amélioration de l'adhésion ou de l'adsorption, il est possible d'utiliser des particules métalliques encapsulées par une enveloppe en polymère organométallique réactif capable de créer des liaisons covalentes inter-particules après évaporation de la phase solvant.

Dans ce cadre, on peut citer de façon non exhaustive, l'encapsulation de particules par un polyméthacrylate à fonctions alcoxysilanes.

Pour plus de détails sur ce procédé, on peut se référer au document suivant « Synthesis and characterization of SiOH functionalized polymer latexes using methacryloxypropyltrimethoxysilane in emulsion polymérisation » (Macro molécules 2002, 35, 6185-6191).

Dans les compositions cosmétiques, les nanoparticules métalliques encapsulées de l'invention sont généralement présentes à une concentration comprise entre 0,0001% et 50%, de préférence entre 0,01% et 5%, et encore plus préférentiellement entre 0,05% et 2% en masse de la masse totale de la composition.

La composition selon l'invention comprend, en outre, un milieu physiologiquement acceptable. On désigne ainsi un milieu susceptible d'être appliqué sur les fibres kératiniques et en particulier sur les cheveux d'êtres humains.

Le milieu physiologiquement acceptable de la composition comprend généralement au moins un solvant, le solvant permet notamment de véhiculer les nanoparticules métalliques encapsulées. Le solvant peut être généralement choisi parmi les solvants organiques, l'eau, et leurs mélanges.

Les solvants organiques sont généralement choisis parmi les alcools aliphatiques en C₁ à C₄ tels que l'éthanol et l'isopropanol, les polyols comme le glycérol ou le propylèneglycol, les alcools aromatiques comme l'alcool benzylique, les alcanes en particulier en C₅ à C₁₀, l'acétone, la méthyl-éthyl-cétone, l'acétate de méthyle, l'acétate de butyle, l'acétate d'alkyle, le diméthoxyéthane, le diéthoxyéthane et leurs mélanges.

Les compositions selon l'invention peuvent être conditionnées sous diverses formes et notamment dans un dispositif aérosol.

La composition selon l'invention peut alors contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Les compositions de l'invention peuvent en outre contenir des additifs cosmétiques usuels choisis parmi cette liste non exhaustive telle que les agents réducteurs, les agents oxydants, les agents épaississants, les adoucissants, les agents anti-mousse, les colorants directs ou d'oxydation, les parfums, les peptisants, les conservateurs, les tensioactifs anioniques ou amphotères, etc.

La composition cosmétique de l'invention peut être une composition cosmétique de traitement en particulier une composition pour apporter de la brillance aux matières kératiniques mais sous une forme de réalisation préférée, il s'agit d'une composition cosmétique capillaire en particulier d'une composition pour apporter aux cheveux de la brillance.

Les compositions cosmétiques capillaires de l'invention après application sur la chevelure peuvent être rincées ou non. Les compositions notamment capillaires (formulations) peuvent se présenter sous différentes formes galéniques tels qu'une lotion, un "spray", une mousse, une laque, un après-shampooing ou un shampooing par exemple.

L'invention sera mieux comprise à l'aide des exemples illustratifs non limitatifs qui suivent et qui constituent des modes de mise en oeuvre préférentiel de la composition de l'invention. Les pourcentages des exemples sont exprimés en poids et m.a. signifie matière active.

### Exemple comparatif

Dans cet exemple, on réalise une composition selon la présente invention, et une composition selon l'art antérieur.

### Composition 1 : Mousse aérosol selon l'invention

| | |
|---|---|
| STARCH ACETATE | 5 % m.a. |
| POLYSORBATE 20 | 0.1% m.a. |
| COCAMIDOPROPYL BETAINE | 0.5% m.a. |
| Nanoparticules d'argent encapsulées ^{[1]} | 1,0% m.a. |
| LAURETH-4 | 0,3% m.a. |
| ISOBUTANE/BUTANE/PROPANE | 5% m.a. |
| Conservateur | qs |
| Parfum | qs |
| Eau | qsp |

- POLYSORBATE 20 : Monolaurate de polyoxyéthylène 20 sorbitan vendu par la société Atlas.
- Tensioactif LAURETH-4 : commercialisé par la société Uniquema.
- Mélange butane-isobutane-propane : 24- 56- 20.

[1] Les nanoparticules coeur-enveloppe « core-shell » sont synthétisées suivant le procédé décrit dans l'exemple n°3 de la demande de brevet WO-A-01/88540. Les coeur en argent de la particule est obtenu par réduction de nitrate d'argent (AgNO₃) par du borohydrure de sodium (NaBH₄). L'encapsulation quant a elle est réalisée par polycondensation en émulsion de tétraéthylorthosilicate (TEOS).

Tels que le montrent les clichés de microscopie électronique à transmission (TEM) réalisés, les particules ainsi encapsulées se présentent sous la forme d'un noyau d'argent natif de 5 à 30 nm de diamètre, recouvert par une couche de silice de 5 nm environ.

### Composition 2 : Mousse aérosol témoin non-conforme à l'invention

| | |
|---|---|
| STARCH ACETATE | 5 % m.a. |
| POLYSORBATE 20 | 0.1% m.a. |
| COCAMIDOPROPYL BETAINE | 0.5% m.a. |
| Nano-particules d'argent non encapsulées ^{[2]} | 1,0% m.a. |
| LAURETH-4 | 0,3% m.a. |
| ISOBUTANE/BUTANE/PROPANE | 5% m.a. |
| Conservateur | qs |
| Parfum | qs |
| Eau | qsp |

- POLYSORBATE 20 : Monolaurate de polyoxyéthylène 20 sorbitan vendu par la société Atlas.
- Tensioactif LAURETH-4 : commercialisé par la société Uniquema.
- Mélange butane-isobutane-propane : 24- 56- 20.

[2] Nanoparticules d'argent commercialisées sous la référence "Colloid Mag" par la société Grant Industries. Tels que le montrent les clichés de microscopie électronique à transmission (TEM) réalisés, les nanoparticules présentent un diamètre compris entre 5 nm et 30 nm.

Chacune des précédentes compositions est appliquée sur une mèche de cheveux châtains de 2,7 g (cheveux européens de 20 cm de longueur) à raison d'un gramme de composition par mèche. Après application, les mèches sont séchées au casque (70°C) pendant 30 minutes.

On réalise ensuite une mesure de brillance sur un lot de 10 mèches traitées comme indiqué précédemment par l'une ou l'autre des compositions.

La brillance est déterminée à l'aide un photogoniomètre par mesure des réflexions spéculaire et diffuse de mèches de cheveux posées à plat sur support. A l'aide d'une lampe xénon à arc de 175Watts (modèle ORC175F) couplé à un filtre V (lambda), une lumière est émise sur la mèche sous un angle de +30° par rapport à la normale de sa surface. A l'aide d'un bras de réception mobile on mesure la réflexion spéculaire (R) correspondant au maximum d'intensité lumineuse réfléchie aux environs de -30° d'angle et la réflexion diffuse (D) correspondant à la lumière réfléchie sous un angle de +15°. Selon l'invention, la brillance est déterminée en calculant le rapport (R)/(D)

Afin d'évaluer la tenue de la brillance dans le temps, la mesure de brillance est renouvelée sur les mêmes mèches traitées après 1 mois de stockage des ces dernières à l'ambiante (20°C et 50% HR).

Les résultats obtenus en terme de stabilité de la brillance sont donnés dans le tableau suivant :

**Tableau**

| | Brillance avant traitement | Brillance juste après traitement | Brillance des mèches traitées après 1 mois |
|---|---|---|---|
| Composition 1 (invention) | 23 ± 3 | 37 ± 1 | 34 ± 3 |
| Composition 2 (art antérieur) | 20 ± 4 | 33 ± 4 | 20 ± 2 |

Comme le montre le précédent tableau, on note que la composition de l'invention conserve ces propriétés de brillance après un mois de stockage des mèches à l'ambiante. Comparativement, la composition de l'art antérieur perd la totalité de son pouvoir réflecteur après 1 mois de stockage des mèches à l'ambiante.

Outre la conservation de la brillance dans le temps, les compositions cosmétiques de l'invention présentent une meilleure stabilité dans le temps vis à vis de l'état de la technique. En effet l'encapsulation limite l'agrégation des nanoparticules métalliques dans les milieux polaires comme l'eau et/ou l'éthanol, permettant ainsi d'obtenir des dispersions colloïdales d'une grande stabilité.

## Revendications

1. Composition cosmétique comprenant, dans un milieu physiologiquement acceptable, au moins un agent présentant une activité cosmétique et des particules comprenant un coeur et une enveloppe solide liée au coeur par une liaison non-covalente, le coeur étant constitué en majorité par au moins un métal, l'enveloppe solide étant constituée par un matériau inorganique, et la taille desdites particules étant inférieure ou égale à 500 nm.

2. Composition selon la revendication 1, dans laquelle l'agent (ou les agents) présentant une activité cosmétique est(sont) choisi(s) parmi :
• les saccharides, les oligosaccharides, les polysaccharides hydrolysés ou non, modifiés ou non,
• les acides aminés, les oligopeptides, les peptides, les protéines hydrolysées ou non, modifiées ou non, les polyacides aminés, les enzymes,
• les acides et alcools gras ramifiés ou non,
• les cires animales, végétales ou minérales,
• les céramides et les pseudo-céramides,
• les acides organiques hydroxylés,
• les filtres UV,
• les antioxydants et les agents anti-radicaux libres,
• les chélatants,
• les agents antipelliculaires,
• les agents régulateurs de séborrée,
• les agents apaisants,
• les tensioactifs cationiques,
• les polymères cationiques, amphotères,
• les silicones organomodifiées ou non,
• les huiles minérales, végétales ou animales,
• les polyisobutènes et poly(α-oléfines),
• les esters,
• les polymères anioniques solubles ou dispersés,
• les polymères non-ioniques solubles ou dispersés,
• les agents réducteurs
• les agents colorants et matières colorantes, et notamment les colorants capillaires,
• les agents moussants,
• les agents filmogènes,
• les particules, autres que les particules à structure coeur-enveloppe définies dans la revendication 1,
• et leurs mélanges.

3. Composition selon l'une quelconque des revendications précédentes dans laquelle l'agent présentant une activité cosmétique est présent dans une proportion de 0,001 à 10 % en poids de la composition cosmétique, de préférence de 0,01 à 5% en poids.

4. Composition cosmétique selon l'une quelconque des revendications précédentes, dans laquelle la taille desdites particules est comprise entre 1 nm et 500 nm, de préférence entre 1 nm et 100 nm, et de préférence encore de 1 nm à 50 nm.

5. Composition cosmétique selon l'une quelconque des revendications précédentes, dans laquelle les particules ont la forme de sphères, lamelles, fibres, tubes, polyèdres ou une forme aléatoire.

6. Composition cosmétique selon l'une quelconque des revendications précédentes, dans laquelle le coeur de la particule est constitué par au moins 80% en masse d'au moins un métal, de préférence par au moins 90% en masse, et de préférence encore par 100% en masse d'au moins un métal.

7. Composition cosmétique selon l'une quelconque des revendications précédentes, dans laquelle le métal qui constitue en majorité le coeur des particules est choisi parmi l'aluminium et les éléments de numéro atomique allant de 21 à 82 et composant les colonnes 3 à 13 de la classification périodique des éléments, et les alliages de ceux-ci.

8. Composition cosmétique selon la revendication 7, dans laquelle le coeur des particules est constitué par un mélange de deux ou plus desdits métaux et/ou alliages de ceux-ci.

9. Composition cosmétique selon l'une quelconque des revendications précédentes, dans laquelle le coeur des particules est un coeur composite constitué de plusieurs zones, des zones adjacentes étant constituées par des métaux, alliages ou mélanges différents.

10. Composition cosmétique selon la revendication 9, dans laquelle le coeur composite des particules est un coeur composite multicouche comprenant un coeur ou noyau interne constitué par un métal, alliage ou mélange de métaux ou d'alliages, recouvert au moins partiellement par une première couche en un métal, alliage de métal ou mélange de métaux ou d'alliages différent de celui constituant le coeur ou noyau interne, et éventuellement par une ou plusieurs autres couches, chacune de ces couches recouvrant au moins partiellement la couche précédente et chacune de ces couches étant constituée par un métal, alliage ou mélange différent de la couche suivante et de la couche précédente.

11. Composition selon l'une quelconque des revendications précédentes, dans laquelle le coeur des particules comprend en outre des impuretés inévitables, et des stabilisants.

12. Composition selon l'une quelconque des revendications précédentes, dans laquelle le coeur des particules comprend en outre des oxydes de métaux.

13. Composition selon l'une quelconque des revendications précédentes, dans laquelle le métal qui constitue en majorité le coeur des particules est choisi parmi les métaux de transition, les métaux des terres rares et leurs alliages et mélanges.

14. Composition selon l'une quelconque des revendications précédentes, dans laquelle le métal qui constitue en majorité le coeur des particules est choisi parmi l'aluminium, le cuivre, l'argent, l'or, l'indium, le fer, le platine, le nickel, le molybdène, le titane, le tungstène, l'antimoine, la palladium, le zinc, l'étain, et leurs alliages et mélanges.

15. Composition selon la revendication 14, dans laquelle le métal qui constitue en majorité le coeur des particules est choisi parmi l'or, l'argent, le palladium, le platine et leurs alliages et mélanges.

16. Composition selon la revendication 15, dans laquelle le métal est l'argent.

17. Composition selon l'une quelconque des revendications précédentes, dans laquelle l'enveloppe est en contact direct avec le métal natif qui constitue en majorité le coeur des particules.

18. Composition selon l'une quelconque des revendications 1 à 16, dans laquelle le coeur est modifié en surface par un traitement modifiant les propriétés de celle-ci.

19. Composition selon la revendication 18, dans laquelle ledit traitement consiste à stabiliser la surface du coeur par une monocouche adsorbée ou liée par covalence.

20. Composition selon l'une quelconque des revendications précédentes, dans laquelle le matériau inorganique qui constitue l'enveloppe des particules est choisi parmi les matériaux constitués par les oxydes métalliques et/ou les polymères organométalliques.

21. Composition selon la revendication 20, dans laquelle les oxydes métalliques sont choisis parmi les oxydes de silicium, de titane, d'aluminium, de zirconium, de zinc, de bore, de lithium, de magnésium, de sodium, de cérium, les oxydes mixtes de ceux-ci, et les mélanges de ces oxydes et oxydes mixtes.

22. Composition selon la revendication 21, dans laquelle l'oxyde métallique est la silice, l'oxyde de titane ou l'alumine.

23. Composition selon la revendication 21, dans laquelle les polymères organométalliques sont choisis parmi les produits de condensation d'alcoxysilanes.

24. Composition selon l'une quelconque des revendications précédentes, dans laquelle l'enveloppe a une épaisseur de 2 à 300 nm, de préférence de 5 à 250 nm, de préférence encore de 10 à 100 nm.

25. Composition selon l'une quelconque des revendications précédentes, dans laquelle l'enveloppe est formée par un procédé physicochimique choisi parmi la séparation de phase ou coacervation et la précipitation contrôlée.

26. Composition selon l'une quelconque des revendications 1 à 24, dans laquelle l'enveloppe est formée par un procédé chimique choisi parmi la polycondensation interfaciale, la polycondensation in-situ et la polymérisation en émulsion.

27. Composition selon l'une quelconque des revendications 1 à 24, dans laquelle le matériau inorganique qui constitue l'enveloppe des particules est choisi parmi les matériaux inorganiques susceptibles d'être obtenus par un procédé sol-gel.

28. Composition selon la revendication 27, dans laquelle le matériau inorganique qui constitue l'enveloppe des particules est choisi parmi les oxydes métalliques et les polymères organométalliques susceptibles d'être obtenus par un procédé sol-gel à partir d'un ou de plusieurs précurseurs.

29. Composition selon la revendication 27, dans laquelle le matériau inorganique qui constitue l'enveloppe des particules est choisi parmi les oxydes métalliques et les polymères organométalliques susceptibles d'être obtenus par polycondensation d'un ou de plusieurs précurseurs alcoxydes métalliques choisis de préférence parmi les alcoxydes de silicium, d'aluminium, de bore, de lithium, de magnésium, de titane, de zirconium et les alcoxydes mixtes de ceux-ci.

30. Composition selon la revendication 28 ou 29, dans laquelle les polymères organométalliques sont choisis parmi les polymères organométalliques susceptibles d'être préparés par polycondensation d'alcoxysilane(s) comprenant un, deux ou trois atomes de silicium et au moins deux fonctions hydroxy ou hydrolysables, tels que méthoxy, éthoxy, propoxy, et comprenant éventuellement en outre des fonctions assurant une compatibilité avec le milieu physiologiquement acceptable et/ou une affinité avec les matières ou fibres kératiniques.

31. Composition selon la revendication 30, dans laquelle les fonctions assurant une compatibilité avec le milieu physiologiquement acceptable et/ou une affinité avec les matières ou fibres kératiniques sont choisies parmi les fonctions améliorant la solubilité dans l'eau telles que les fonctions alkyl amine, alkyl alcool, alkyl thiol, alkyl acide, alkyl polyamine, alkyl polyol et alkyl polycarboxylique.

32. Composition selon la revendication 30 ou 31, dans laquelle lesdits alcoxysilanes sont choisis parmi le 3-aminopropyltriéthoxysilane, le 3-aminopropyl-méthyl-diéthoxysilane, le 3-[bis(hydroxyéthyl)aminopropyl]triéthoxysilane.

33. Composition selon l'une quelconque des revendications 28 à 31, dans laquelle le précurseur du procédé sol-gel est le tétraéthylorthosilicate (TEOS).

34. Composition selon l'une quelconque des revendications 1 à 24, dans laquelle les particules incluses dans les compositions de l'invention sont susceptibles d'être préparées par un procédé dans lequel on réalise, dans un milieu aqueux, la condensation sur des particules constituées en majorité par au moins un métal, lesdites particules étant destinées à former le coeur des particules coeur-enveloppe que l'on souhaite obtenir, de composés organiques du silicium soluble dans l'eau, peu ou pas polymérisés, choisis parmi les organosilanes comportant un atome de silicium et les organosiloxanes comportant deux ou trois atomes de silicium, ces composés organiques du silicium comportant en outre au moins une fonction chimique basique et au moins deux groupes hydrolysables ou hydroxyles par molécule, ces composés organiques du silicium, peu ou pas polymérisés, étant neutralisés à raison de 1/1000 à 99/100, de préférence de 0,2/100 à 70/100 par un agent de neutralisation.

35. Composition selon l'une quelconque des revendications 1 à 24, dans laquelle les particules enrobées, encapsulées incluses dans les compositions de l'invention sont susceptibles d'être préparées par un procédé dans lequel on réalise, dans un milieu aqueux, la condensation sur des particules constituées en majorité par au moins un métal, lesdites particules étant destinées à former le coeur de la particule « enrobée » coeur-enveloppe que l'on souhaite obtenir, de composés organiques du silicium solubles dans l'eau peu ou pas polymérisés, choisis parmi les organosilanes comportant un atome de silicium et les organosiloxanes comportant deux ou trois atomes de silicium, ces composés organiques du silicium comportant par molécule en outre au moins deux groupes hydroxyles ou deux groupes fonctionnels hydrolysables et au moins deux groupes fonctionnels non-hydrolysables, un au moins de ces groupes fonctionnels non-hydrolysables étant un groupe fonctionnel ayant un effet cosmétique et au moins un autre de ces groupes fonctionnels non-hydrolysables étant un groupe fonctionnel solubilisant.

36. Composition selon la revendication 35, dans laquelle le ou les groupe(s) à effet cosmétique est(sont) un(des) groupe(s) à fonction colorante, de protection anti-UV, anti-bactérienne ou anti-fongique ou à fonction réductrice.

37. Composition selon l'une quelconque des revendications 1 à 24, dans laquelle les les particules enrobées, encapsulées incluses dans les compositions de l'invention sont susceptibles d'être préparées par un procédé dans lequel on réalise, dans un milieu aqueux, la condensation sur des particules constituées en majorité par au moins un métal natif, lesdites particules étant destinées à former le coeur de la particule coeur-enveloppe que l'on souhaite obtenir, de composés organiques du silicium solubles dans l'eau, peu ou pas polymérisés, choisis parmi les organosilanes comportant un atome de silicium et les organosiloxanes comportant deux ou trois atomes de silicium, ces composés organiques du silicium comportant en outre au moins une fonction chimique solubilisante non basique et au moins deux groupes hydrolysables par molécule.

38. Composition selon l'une quelconque des revendications 1 à 24, dans laquelle les particules enrobées, encapsulées, incluses dans les compositions de l'invention sont susceptibles d'être préparées par un procédé comprenant les étapes suivantes :
a) on prépare un mélange
(i) d'une dispersion de particules constituées en majorité par au moins un métal natif et destinées à former le coeur de la particule coeur-enveloppe que l'on souhaite obtenir, dans un milieu liquide, notamment un milieu aqueux ou alcoolique ou huileux, éventuellement en présence d'un dispersant,
(ii) et d'une solution d'un matériau hybride organo-minéral réticulé, ledit matériau (avant hydrolyse) étant obtenu par voie sol-gel à partir d'un prémélange comprenant:
(A) au moins un composé métallique ou métallo-organique, et
(B) au moins un polymère organique fonctionnalisé ou un précurseur de celui-ci, ou au moins un polymère siliconé fonctionnalisé ou un précurseur de celui-ci, ce dernier étant différent de (A) ,
b) ledit mélange est mis en contact avec de l'eau lorsque ladite dispersion des particules destinées à former le coeur est une dispersion dans un milieu liquide alcoolique ou huileux.

39. Composition selon l'une quelconque des revendications précédentes, dans laquelle la surface externe des particules est modifiée de façon covalente par au moins un groupement chimique qui est susceptible d'améliorer l'adsorption des particules sur les matières kératiniques telles que les cheveux.

40. Composition selon l'une quelconque des revendications précédentes, dans laquelle la surface externe des particules est modifiée de façon covalente par au moins un groupement chimique susceptible de réagir chimiquement avec les matières kératiniques telles que les cheveux.

41. Composition selon la revendication 39, dans laquelle le groupement chimique susceptible d'améliorer l'adsorption des particules sur les matières kératiniques est choisi parmi les groupements suivants :
- Acides carboxyliques et leurs sels,
- Amines primaires, secondaires, tertiaires et quaternaires,
- Phosphates,
- Oxydes de soufre tels que sulfones, sulfoniques, sulfoxides, et sulfates,
- Noyaux aromatiques tels que phényle, triazine, thiophène et imidazole.

42. Composition selon la revendication 40, dans laquelle le groupement chimique susceptible de réagir chimiquement avec les matières kératiniques est choisi parmi les groupements suivants :
- Epoxydes,
- Vinyle et vinyle activé : acrylonitrile, esters acrylique et méthacrylique, acide et esters crotonique, acide et esters cinnamique, styrène et dérivés, butadiène, éthers de vinyle, vinyle cétone, esters maléique, maléimides, vinyl sulfones, ...
- Acides carboxyliques et leurs dérivés : anhydride, chlorure d'acide, esters,
- Acétals, hémi-acétals,
- Aminals, hémi-aminals,
- Cétones et alpha-hydroxycétones, alpha-halocétones,
- Lactones, thiolactones,
- Isocyanates,
- Thiocyanates,
- Imines,
- Imides tels que succinimides, et glutimides,
- Pyridyldithio,
- N hydroxysuccinimide esters,
- Imidates,
- Oxazine et oxazoline,
- Oxazinium et oxazolinium,
- Groupements de formule R₁X, dans laquelle R₁ est un groupe alkyle de 1 à 30C, un groupe aryle comprenant de 6 à 30C, un groupe aralkyle de 7 à 30C (groupe alkyle de 1 à 30C), et X est un groupe partant tel que I, Br, Cl, OSO₃R où R est H, un groupe alkyle de 1 à 30C, -SO₂R' où R' est H, un groupe alkyle de 1 à 30C, un groupe tosyle, N(R")₃ où R" est un groupe alkyle de 1 à 30C, OPO₃(R''')₂ où R"' est H ou un groupe alkyle de 1 à 30C ; des groupements de formule R₁X préférés sont les halogénures d'alkyle, d'aryle ou d'aralkyle ;
- Groupements de formule R₂X où R₂ représente un cycle carboné de 3 à 30C ou un hétérocycle insaturé de 3 à 20 chaînons comprenant un ou plusieurs hétéroatomes choisis parmi N, S, O et P, et X est un groupe partant tel que défini ci-dessus ; des groupements de formule R₂X préférés sont les halogénures de cycles insaturés tels que la chlorotriazine, la chloropyrimidine, la chloroquinoxaline, et le chlorobenzotriazole,
- Groupements de formule : R₃SO₂X où R₃ a la même signification que R₁ et X représente un groupe partant et a la signification déjà donnée ci-dessus,
- Lactones,
- Thiolactones,
- Siloxanes.

43. Composition selon l'une quelconque des revendications 1 à 38, dans laquelle l'enveloppe des particules est constituée par un polymère organométallique réactif capable de créer des liaisons covalentes inter-particules.

44. Composition selon l'une quelconque des revendications précédentes, dans laquelle les particules sont présentes dans la composition à une concentration comprise entre 0,0001% et 50%, de préférence entre 0,01 et 5% et de préférence encore entre 0,05% et 2% en masse de la masse totale de la composition.

45. Composition selon l'une quelconque des revendications précédentes, dans laquelle le milieu physiologiquement acceptable comprend au moins un solvant.

46. Composition selon la revendication 45, dans laquelle le solvant est choisi parmi les solvants organiques, l'eau, et leurs mélanges.

47. Composition selon la revendication 46, dans laquelle les solvants organiques sont choisis parmi les alcools aliphatiques en C₁ à C₄ tels que l'éthanol et l'isopropanol, les polyols comme le glycérol ou le propylèneglycol, les alcools aromatiques comme l'alcool benzylique, les alcanes en particulier en C₅ à C₁₀, l'acétone, la méthyl-éthyl-cétone, l'acétate de méthyle, l'acétate de butyle, l'acétate d'alkyle, le diméthoxyéthane, le diéthoxyéthane et leurs mélanges.

48. Composition selon l'une quelconque des revendications précédentes qui contient en outre des additifs cosmétiques choisis parmi les agents réducteurs, les agents oxydants, les agents épaississants, les adoucissants, les agents anti-mousse, les colorants directs ou d'oxydation, les parfums, les peptisants, les conservateurs, les tensioactifs, anioniques ou amphotères.

49. Composition selon l'une quelconque des revendications précédentes, qui est une composition cosmétique de traitement, en particulier pour apporter de la brillance aux matières kératiniques.

50. Composition selon la revendication 49, qui est une composition capillaire, en particulier pour apporter aux cheveux de la brillance.

51. Composition selon la revendication 50, qui se présente sous la forme d'une lotion, d'un "spray", d'une mousse, d'une laque, d'un après-shampooing ou d'un shampooing.

52. Composition selon l'une quelconque des revendications précédentes qui est conditionnée dans un dispositif aérosol.

53. Procédé cosmétique de traitement des matières kératiniques telles que les cheveux, en particulier pour apporter de la brillance aux matières kératiniques telles que les cheveux, comprenant l'application sur des matières kératiniques tels que les cheveux d'une composition selon l'une quelconque des revendications 1 à 52.

54. Utilisation de la composition selon l'une quelconque des revendications 1 à 52, pour apporter de la brillance à des matières kératiniques telles que les cheveux.

55. Utilisation des particules telles que décrites dans l'une quelconque des revendications 1 à 52 dans une composition cosmétique pour apporter de la brillance aux matières kératiniques telles que les cheveux.

## Claims

1. Cosmetic composition comprising, in a physiologically acceptable medium, at least one agent exhibiting a cosmetic activity and particles comprising a core and a solid shell bonded to the core via a noncovalent bond, the core being predominantly composed of at least one metal, the solid shell being composed of an inorganic material, and the size of the said particles being less than or equal to 500 nm.

2. Composition according to Claim 1, in which the agent (or agents) exhibiting a cosmetic activity is (are) chosen from:
• saccharides, oligosaccharides or polysaccharides which may or may not be hydrolysed and which may or may not be modified,
• amino acids, oligopeptides, peptides, proteins, which may or may not be hydrolysed and which may or may not be modified, poly(amino acid)s or enzymes,
• branched or unbranched fatty acids and alcohols,
• animal, vegetable or mineral waxes,
• ceramides and pseudoceramides,
• hydroxylated organic acids,
• UV screening agents,
• antioxidants and agents for combating free radicals,
• chelating agents,
• antidandruff agents,
• seborrhoea-regulating agents,
• soothing agents,
• cationic surfactants,
• cationic or amphoteric polymers,
• organomodified or nonorganomodified silicones,
• mineral, vegetable or animal oils,
• polyisobutenes and poly(α-olefin)s,
• esters,
• soluble or dispersed anionic polymers,
• soluble or dispersed non-ionic polymers,
• reducing agents,
• colouring agents and colouring materials, and in particular hair dyes,
• foaming agents,
• film-forming agents,
• particles, other than the particles having a core-shell structure defined in Claim 1,
• and their mixtures.

3. Composition according to either one of the preceding claims, in which the agent exhibiting a cosmetic activity is present in a proportion of 0.001 to 10% by weight of the cosmetic composition, preferably of 0.01 to 5% by weight.

4. Cosmetic composition according to any one of the preceding claims, in which the size of the said particles is between 1 nm and 500 nm, preferably between 1 nm and 100 nm and more preferably from 1 nm to 50 nm.

5. Cosmetic composition according to any one of the preceding claims, in which the particles have the shape of spheres, flakes, fibres, tubes or polyhedra or a random shape.

6. Cosmetic composition according to any one of the preceding claims, in which the core of the particle is composed of at least 80% by weight of at least one metal, preferably of at least 90% by weight and more preferably of 100% by weight of at least one metal.

7. Cosmetic composition according to any one of the preceding claims, in which the metal of which the core of the particles is predominantly composed is chosen from aluminium and the elements with an atomic number ranging from 21 to 82 and composing Groups 3 to 13 of the Periodic Table of the Elements, and the alloys of the latter.

8. Cosmetic composition according to Claim 7, in which the core of the particles is composed of a mixture of two or more of the said metals and/or alloys of the latter.

9. Cosmetic composition according to any one of the preceding claims, in which the core of the particles is a composite core composed of several regions, adjacent regions being composed of different metals, alloys or mixtures.

10. Cosmetic composition according to Claim 9, in which the composite core of the particles is a multilayer composite core comprising an inner core composed of a metal, alloy or mixture of metals or alloys, at least partially covered by a first layer made of a metal, metal alloy or mixture of metals or alloys which is different from that constituting the inner core and optionally by one or more other layers, each of these layers at least partially covering the preceding layer and each of these layers being composed of a metal, alloy or mixture of alloys which is different from the following layer and from the preceding layer.

11. Composition according to any one of the preceding claims, in which the core of the particles additionally comprises stabilizers and unavoidable impurities.

12. Composition according to any one of the preceding claims, in which the core of the particles additionally comprises metal oxides.

13. Composition according to any one of the preceding claims, in which the metal of which the core of the particles is predominantly composed is chosen from transition metals, rare earth metals and their alloys and mixtures.

14. Composition according to any one of the preceding claims, in which the metal of which the core of the particles is predominantly composed is chosen from aluminium, copper, silver, gold, indium, iron, platinum, nickel, molybdenum, titanium, tungsten, antimony, palladium, zinc, tin and their alloys and mixtures.

15. Composition according to Claim 14, in which the metal of which the core of the particles is predominantly composed is chosen from gold, silver, palladium, platinum and their alloys and mixtures.

16. Composition according to Claim 15, in which the metal is silver.

17. Composition according to any one of the preceding claims, in which the shell is in direct contact with the native metal of which the core of the particles is predominantly composed.

18. Composition according to any one of Claims 1 to 16, in which the core is modified at the surface by a treatment which modifies the properties of the latter.

19. Composition according to Claim 18, in which the said treatment consists in stabilizing the surface of the core by an adsorbed or covalently bonded monolayer.

20. Composition according to any one of the preceding claims, in which the inorganic material which constitutes the shell of the particles is chosen from materials composed of metal oxides and/or organometallic polymers.

21. Composition according to Claim 20, in which the metal oxides are chosen from silicon, titanium, aluminium, zirconium, zinc, boron, lithium, magnesium, sodium and cerium oxides, the mixed oxides of the latter, and the mixtures of these oxides and mixed oxides.

22. Composition according to Claim 21, in which the metal oxide is silica, titanium oxide or alumina.

23. Composition according to Claim 21, in which the organometallic polymers are chosen from the condensation products of alkoxysilanes.

24. Composition according to any one of the preceding claims, in which the shell has a thickness of 2 to 300 nm, preferably of 5 to 250 nm, more preferably of 10 to 100 nm.

25. Composition according to any one of the preceding claims, in which the shell is formed by a physicochemical process chosen from phase separation or coacervation and controlled precipitation.

26. Composition according to any one of Claims 1 to 24, in which the shell is formed by a chemical process chosen from interfacial polycondensation, in situ polycondensation and emulsion polymerization.

27. Composition according to any one of Claims 1 to 24, in which the inorganic material which constitutes the shell of the particles is chosen from inorganic materials capable of being obtained by a sol-gel process.

28. Composition according to Claim 27, in which the inorganic material which constitutes the shell of the particles is chosen from metal oxides and organometallic polymers capable of being obtained by a sol-gel process from one or more precursors.

29. Composition according to Claim 27, in which the inorganic material which constitutes the shell of the particles is chosen from metal oxides and organometallic polymers capable of being obtained by polycondensation of one or more metal alkoxide precursors preferably chosen from silicon, aluminium, boron, lithium, magnesium, titanium and zirconium alkoxides, and the mixed alkoxides thereof.

30. Composition according to Claim 28 or 29, in which the organometallic polymers are chosen from organometallic polymers capable of being prepared by polycondensation of alkoxysilane(s) comprising one, two or three silicon atoms and at least two hydroxyl or hydrolysable functional groups, such as methoxy, ethoxy or propoxy, and optionally comprising, in addition, functional groups which render compatible with the physiologically acceptable medium and/or which contribute an affinity with keratinous substances or fibres.

31. Composition according to Claim 30, in which the functional groups which render compatible with the physiologically acceptable medium and/or which contribute an affinity with keratinous substances or fibres are chosen from functional groups which improve the solubility in water, such as alkyl amine, alkyl alcohol, alkyl thiol, alkyl acid, alkyl polyamine, alkyl polyol and alkyl polycarboxyl functional groups.

32. Composition according to Claim 30 or 31, in which the said alkoxysilanes are chosen from 3-aminopropyltriethoxysilane, (3-aminopropyl)methyldiethoxysilane or {3-[bis(hydroxyethyl)amino]propyl}triethoxysilane.

33. Composition according to any one of Claims 28 to 31, in which the precursor of the sol-gel process is tetraethyl orthosilicate (TEOS).

34. Composition according to any one of Claims 1 to 24, in which the particles included in the compositions of the invention are capable of being prepared by a process in which water-soluble organic silicon compounds, which are not polymerized or which are polymerized only to a slight extent and which are chosen from organosilanes comprising a silicon atom and organosiloxanes comprising two or three silicon atoms, are condensed, in an aqueous medium, on particles predominantly composed of at least one metal, the said particles being intended to form the core of the core-shell particles which it is desired to obtain, these organic silicon compounds additionally comprising at least one basic chemical functional group and at least two hydrolysable or hydroxyl groups per molecule, these organic silicon compounds, which are not polymerized or which are polymerized only to a slight extent, being neutralized in a proportion of 1/1000 to 99/100, preferably of 0.2/100 to 70/100, by a neutralizing agent.

35. Composition according to any one of Claims 1 to 24, in which the coated or encapsulated particles included in the compositions of the invention are capable of being prepared by a process in which water-soluble organic silicon compounds, which are not polymerized or which are polymerized only to a slight extent and which are chosen from organosilanes comprising a silicon atom and organosiloxanes comprising two or three silicon atoms, are condensed, in an aqueous medium, on particles predominantly composed of at least one metal, the said particles being intended to form the core of the "coated" core-shell particle which it is desired to obtain, these organic silicon compounds additionally comprising, per molecule, at least two hydroxyl groups or two hydrolysable functional groups and at least two non-hydrolysable functional groups, at least one of these non-hydrolysable functional groups being a functional group having a cosmetic effect and at least one other of these non-hydrolysable functional groups being a solubilizing functional group.

36. Composition according to Claim 35, in which the group(s) having a cosmetic effect is(are) (a) group(s) having a colouring, UV screening, bactericidal or fungicidal function or having a reducing function.

37. Composition according to any one of Claims 1 to 24, in which the coated or encapsulated particles included in the compositions of the invention are capable of being prepared by a process in which water-soluble organic silicon compounds, which are not polymerized or which are polymerized only to a slight extent and which are chosen from organosilanes comprising a silicon atom and organosiloxanes comprising two or three silicon atoms, are condensed, in an aqueous medium, on particles predominantly composed of at least one native metal, the said particles being intended to form the core of the core-shell particle which it is desired to obtain, these organic silicon compounds additionally comprising at least one non-basic solubilizing chemical functional group and at least two hydrolysable groups per molecule.

38. Composition according to any one of Claims 1 to 24, in which the coated or encapsulated particles included in the compositions of the invention are capable of being prepared by a process comprising the following stages:
a) the preparation is carried out of a mixture
(i) of a dispersion in a liquid medium, in particular an aqueous or alcoholic or oily medium, optionally in the presence of a dispersant, of particles predominantly composed of at least one native metal and intended to form the core of the core-shell particle which it is desired to obtain,
(ii) and of a solution of a crosslinked hybrid organic/inorganic material, the said material (before hydrolysis) being obtained by the sol-gel route from a premix comprising:
(A) at least one metal compound or organometallic compound, and
(B) at least one functionalized organic polymer or precursor of this polymer or at least one functionalized silicone polymer or a precursor of this polymer, the latter being different from (A),
b) the said mixture is brought into contact with water when the said dispersion of the particles intended to form the core is a dispersion in an alcoholic or oily liquid medium.

39. Composition according to any one of the preceding claims, in which the outer surface of the particles is covalently modified by at least one chemical group which is capable of improving the adsorption of the particles on keratinous substances, such as the hair.

40. Composition according to any one of the preceding claims, in which the outer surface of the particles is covalently modified by at least one chemical group capable of reacting chemically with keratinous substances, such as the hair.

41. Composition according to Claim 39, in which the chemical group capable of improving the adsorption of the particles on keratinous substances is chosen from the following groups:
- Carboxylic acids and their salts,
- Primary, secondary, tertiary and quaternary amines,
- Phosphates,
- Sulphur oxides, such as sulphones, sulphonic, sulphoxides and sulphates,
- Aromatic rings, such as phenyl, triazine, thiophene and imidazole.

42. Composition according to Claim 40, in which the chemical group capable of reacting chemically with keratinous substances is chosen from the following groups:
- Epoxides,
- Vinyl and activated vinyl: acrylonitrile, acrylic and methacrylic esters, crotonic acid and esters, cinnamic acid and esters, styrene and derivatives, butadiene, vinyl ethers, vinyl ketones, maleic esters, maleimides, vinyl sulphones, and the like,
- Carboxylic acids and their derivatives: anhydride, acid chloride, esters,
- Acetals, hemiacetals,
- Aminals, hemiaminals,
- Ketones and α-hydroxyketones, α-haloketones,
- Lactones, thiolactones,
- Isocyanates,
- Thiocyanates,
- Imines,
- Imides, such as succinimides and glutimides,
- Pyridyldithio,
- N-Hydroxysuccinimide esters,
- Imidates,
- Oxazine and oxazoline,
- Oxazinium and oxazolinium,
- Groups of formula R₁X in which R₁ is an alkyl group of 1 to 30C, an aryl group comprising from 6 to 30C or an aralkyl group of 7 to 30C (alkyl group of 1 to 30C) and X is a leaving group such as I, Br, Cl, OSO₃R, where R is H or an alkyl group of 1 to 30C, -SO₂R', where R' is H or an alkyl group of 1 to 30C, a tosyl group, N(R")₃, where R" is an alkyl group of 1 to 30C, or OPO₃R"'₂, where R"' is H or an alkyl group of 1 to 30C; preferred groups of formula R₁X are alkyl, aryl or aralkyl halides;
- Groups of formula R₂X where R₂ represents a carbon ring of 3 to 30C or an unsaturated heterocycle with 3 to 20 ring members comprising one or more heteroatoms chosen from N, S, O and P, and X is a leaving group as defined above; preferred groups of formula R₂X are the halides of unsaturated rings, such as chlorotriazine, chloropyrimidine, chloroquinoxaline and chlorobenzotriazole,
- Groups of formula R₃SO₂X, where R₃ has the same meaning as R₁ and X represents a leaving group and has the meaning already given above,
- Lactones,
- Thiolactones,
- Siloxanes.

43. Composition according to any one of Claims 1 to 38, in which the shell of the particles is composed of a reactive organometallic polymer capable of creating interparticle covalent bonds.

44. Composition according to any one of the preceding claims, in which the particles are present in the composition at a concentration of between 0.0001% and 50%, preferably between 0.01% and 5% and more preferably between 0.05% and 2% by weight of the total weight of the composition.

45. Composition according to any one of the preceding claims, in which the physiologically acceptable medium comprises at least one solvent.

46. Composition according to Claim 45, in which the solvent is chosen from organic solvents, water and their mixtures.

47. Composition according to Claim 46, in which the organic solvents are chosen from C₁ to C₄ aliphatic alcohols, such as ethanol and isopropanol, polyols, such as glycerol or propylene glycol, aromatic alcohols, such as benzyl alcohol, alkanes, in particular C₅ to C₁₀ alkanes, acetone, methyl ethyl ketone, methyl acetate, butyl acetate, alkyl acetate, dimethoxyethane, diethoxyethane and their mixtures.

48. Composition according to any one of the preceding claims, which additionally comprises cosmetic additives chosen from reducing agents, oxidizing agents, thickening agents, softeners, antifoaming agents, direct or oxidation dyes, fragrances, peptizing agents, preservatives, or anionic or amphoteric surfactants.

49. Composition according to any one of the preceding claims, which is a cosmetic treatment composition, in particular for contributing sheen to keratinous substances.

50. Composition according to Claim 49, which is a hair composition, in particular for contributing sheen to the hair.

51. Composition according to Claim 50, which is provided in the form of a lotion, of a spray, of a foam, of a lacquer, of a conditioner or of a shampoo.

52. Composition according to any one of the preceding claims, which is packaged in an aerosol device.

53. Cosmetic process for the treatment of keratinous substances, such as the hair, in particular for contributing sheen to keratinous substances, such as the hair, comprising the application to keratinous substances, such as the hair, of a composition according to any one of Claims 1 to 52.

54. Use of the composition according to any one of Claims 1 to 52 for contributing sheen to keratinous substances, such as the hair.

55. Use of the particles as described in any one of Claims 1 to 52 in a cosmetic composition for contributing sheen to keratinous substances, such as the hair.

## Patentansprüche

1. Kosmetische Zusammensetzung, die in einem physiologisch akzeptablen Medium, mindestens einen Stoff, der eine kosmetische Wirksamkeit aufweist, und Partikel enthält, die einen Kern und eine feste Schale aufweisen, die an den Kern über eine nichtkovalente Bindung gebunden ist, wobei der Kern überwiegend aus mindestens einem Metall und die feste Schale aus einem anorganischen Material gebildet wird und wobei die Größe der genannten Partikel kleiner oder gleich 500 nm ist.

2. Zusammensetzung nach Anspruch 1, wobei der (oder die) Stoffe mit einer kosmetischen Wirksamkeit aufweist, ausgewählt ist (oder sind) unter:
• Sacchariden, Oligosacchariden, Polysacchariden, die hydrolysiert oder nicht hydrolysiert, modifiziert oder nicht modifiziert sind,
• Aminosäuren, Oligopeptiden, Peptiden, Proteinen, die hydrolysiert oder nicht hydrolysiert, modifiziert oder nicht modifiziert sind, Polyaminosäuren, Enzymen,
• verzweigten oder unverzweigten Fettsäuren und Fettalkoholen,
• tierischen, pflanzlichen oder mineralischen Wachsen,
• Ceramiden und Pseudoceramiden,
• hydroxylierten organischen Säuren,
• UV-Filtern,
• Antioxidantien und Radikalfängern für freie Radikale,
• Chelatbildnern,
• Antischuppenmitteln,
• Seborrhoe regulierenden Mitteln,
• beruhigenden Stoffen,
• kationischen grenzflächenaktiven Stoffen,
• kationischen, amphoteren Polymeren,
• Siliconen, die organomodifiziert oder nicht organomodifiziert sind,
• mineralischen, pflanzlichen oder tierischen Ölen,
• Polyisobutenen und Poly-α-olefinen,
• Estern,
• löslichen oder dispergierten anionischen Polymeren,
• löslichen oder dispergierten nichtionischen Polymeren,
• Reduktionsmitteln,
• Färbemitteln und Farbstoffen, insbesondere Haarfärbemitteln,
• Schaumbildnern,
• filmbildenden Stoffen,
• Partikeln, die von den in Anspruch 1 definierten Partikeln mit Kern-Schale-Struktur verschieden sind,
• und deren Gemischen.

3. Zusammensetzung nach einem der vorhergehenden Ansprüche, wobei der Stoff, der eine kosmetische Aktivität aufweist, in einem Mengenanteil von 0,001 bis 10 Gew.-% der kosmetischen Zusammensetzung und vorzugsweise von 0,01 bis 5 Gew.-% enthalten ist.

4. Kosmetische Zusammensetzung nach einem der vorhergehenden Ansprüche, wobei die Größe der genannten Partikel im Bereich von 1 bis 500 nm, vorzugsweise im Bereich von 1 bis 100 nm und noch bevorzugter im Bereich von 1 bis 50 nm liegt.

5. Kosmetische Zusammensetzung nach einem der vorhergehenden Ansprüche, wobei die Partikel die Gestalt von Sphären, Lamellen, Fasern, Röhren, Polyedern oder eine zufällige Gestalt haben.

6. Kosmetische Zusammensetzung nach einem der vorhergehenden Ansprüche, wobei der Kern des Partikels aus mindestens 80 Masse-% mindestens eines Metalls, vorzugsweise aus mindestens 90 Masse-% und noch bevorzugter aus 100 Masse-% mindestens eines Metalls gebildet ist.

7. Kosmetische Zusammensetzung nach einem der vorhergehenden Ansprüche, wobei das Metall, das überwiegend den Kern der Partikel bildet, ausgewählt ist unter Aluminium und den Elementen, die eine Ordnungszahl von 21 bis 82 aufweisen und in der Klassifikation des Periodensystems der Elemente die Spalten 3 bis 13 bilden, und deren Legierungen.

8. Kosmetische Zusammensetzung nach Anspruch 7, wobei der Kern der Partikel aus einem Gemisch aus zwei oder mehreren der genannten Metalle und/ oder deren Legierungen gebildet ist.

9. Kosmetische Zusammensetzung nach einem der vorhergehenden Ansprüche, wobei der Kern der Partikel ein aus mehreren Zonen gebildeter Verbundkern ist, wobei benachbarte Zonen aus unterschiedlichen Metallen, Legierungen oder Gemischen gebildet werden.

10. Kosmetische Zusammensetzung nach Anspruch 9, wobei der Verbundkern der Partikel ein vielschichtiger Verbundkern ist, der einen Kern oder inneren Nukleus enthält, welcher aus einem Metall, einer Legierung oder einem Gemisch aus Metallen oder Legierungen gebildet ist, der mindestens teilweise von einer ersten Schicht eines Metalls, einer Metalllegierung oder eines Gemisches aus Metallen oder Legierungen bedeckt ist, das von demjenigen verschieden ist, das den Kern oder inneren Nukleus bildet, und gegebenenfalls durch eine oder mehrere weitere Schichten bedeckt ist, wobei jede dieser Schichten ihre vorhergehende Schicht zumindest teilweise bedeckt und jede dieser Schichten aus einem Metall, einer Legierung oder einem Gemisch gebildet wird, das anders als das der folgenden und der vorherigen Schicht ist.

11. Zusammensetzung nach einem der vorhergehenden Ansprüche, wobei der Kern der Partikel ferner unvermeidbare Verunreinigungen und Stabilisierungsmittel enthält.

12. Zusammensetzung nach einem der vorhergehenden Ansprüche, wobei der Kern der Partikel ferner Metalloxide enthält.

13. Zusammensetzung nach einem der vorhergehenden Ansprüche, wobei das Metall, das überwiegend den Kern der Partikel bildet, ausgewählt ist unter den Übergangsmetallen, den Seltenerdmetallen und deren Legierungen und Gemischen.

14. Zusammensetzung nach einem der vorhergehenden Ansprüche, wobei das Metall, das überwiegend den Kern der Partikel bildet, ausgewählt ist unter Aluminium, Kupfer, Silber, Gold, Indium, Eisen, Platin, Nickel, Molybdän, Titan, Wolfram, Antimon, Palladium, Zink, Zinn und deren Legierungen und Gemischen.

15. Zusammensetzung nach Anspruch 14, wobei das Metall, das überwiegend den Kern der Partikel bildet, ausgewählt ist unter Gold, Silber, Palladium, Platin und deren Legierungen und Gemischen.

16. Zusammensetzung nach Anspruch 15, wobei das Metall Silber ist.

17. Zusammensetzung nach einem der vorhergehenden Ansprüche, wobei die Schale in direktem Kontakt mit dem gediegenen Metall ist, das überwiegend den Kern der Partikel bildet.

18. Zusammensetzung nach einem der Ansprüche 1 bis 16, wobei der Kern an der Oberfläche durch eine Behandlung, die deren Eigenschaften verändert, modifiziert ist.

19. Zusammensetzung nach Anspruch 18, wobei die genannte Behandlung daraus besteht, die Oberfläche des Kerns durch eine adsorbierte oder kovalent gebundene Monoschicht zu stabilisieren.

20. Zusammensetzung nach einem der vorhergehenden Ansprüche, wobei das anorganische Material, das die Schale der Partikel bildet, unter den Materialien ausgewählt ist, die aus Metalloxiden und/oder Metall-organischen Polymeren gebildet werden.

21. Zusammensetzung nach Anspruch 20, wobei die Metalloxide unter den Oxiden des Siliciums, Titans, Aluminiums, Zirkons, Zinks, Bors, Lithiums, Magnesiums, Natriums, Cers, deren Mischoxiden und Gemischen dieser Oxide und Mischoxide ausgewählt sind.

22. Zusammensetzung nach Anspruch 21, wobei das Metalloxid Siliciumdioxid, Titanoxid oder Aluminiumoxid ist.

23. Zusammensetzung nach Anspruch 21, wobei die Metall-organischen Polymere unter den Kondensationsprodukten von Alkoxysilanen ausgewählt sind.

24. Zusammensetzung nach einem der vorhergehenden Ansprüche, wobei die Schale eine Dicke von 2 bis 300 nm, vorzugsweise von 5 bis 250 nm und noch bevorzugter von 10 bis 100 nm aufweist.

25. Zusammensetzung nach einem der vorhergehenden Ansprüche, wobei die Schale durch ein physikalisch-chemisches Verfahren hergestellt ist, das ausgewählt ist unter Phasentrennung oder Koazervation und kontrollierter Präzipitierung.

26. Zusammensetzung nach einem der Ansprüche 1 bis 24, wobei die Schale durch ein chemisches Verfahren hergestellt ist, das unter Grenzflächen-Polykondensation, in situ Polykondensation und Emulsionspolymerisation ausgewählt ist.

27. Zusammensetzung nach einem der Ansprüche 1 bis 24, wobei das anorganische Material, das die Schale der Partikel bildet, unter den anorganischen Materialien ausgewählt ist, die durch ein Sol-Gel-Verfahren erhältlich sind.

28. Zusammensetzung nach Anspruch 27, wobei das anorganische Material, das die Schale der Partikel bildet, unter den Metalloxiden und den Metall-organischen Polymeren ausgewählt ist, die durch ein Sol-Gel-Verfahren ausgehend von einem oder mehreren Vorläufern erhältlich sind.

29. Zusammensetzung nach Anspruch 27, wobei das anorganische Material, das die Schale der Partikel bildet, unter den Metalloxiden und den Metall-organischen Polymeren ausgewählt ist, die durch Polykondensation einer oder mehrerer Metallalkoxid-Vorläufer, die vorzugsweise ausgewählt sind unter den Alkoxiden des Siliciums, Aluminiums, Bors, Lithiums, Magnesiums, Titans, Zirconiums und deren gemischten Alkoxiden, erhältlich sind.

30. Zusammensetzung nach Anspruch 28 oder 29, wobei die Metall-organischen Polymere unter den Metall-organischen Polymeren ausgewählt sind, die durch Polykondensation von Alkoxysilan(en) erhältlich sind, welche ein, zwei oder drei Siliciumatome und mindestens zwei Hydroxyfunktionen oder hydrolysierbare Funktionen, wie Methoxy, Ethoxy, Propoxy, enthalten und die gegebenenfalls ferner Funktionen enthalten, die für eine Kompatibilität mit dem physiologisch akzeptablen Medium und/oder für eine Affinität mit den Keratinsubstanzen oder Keratinfasern sorgen.

31. Zusammensetzung nach Anspruch 30, wobei die Funktionen, die für eine Kompatibilität mit dem physiologisch akzeptablen Medium und/oder für eine Affinität mit den Keratinsubstanzen oder Keratinfasern sorgen, unter solchen Funktionen ausgewählt sind, die die Löslichkeit in Wasser verbessern, wie die Funktionen Alkylamin, Alkylalkohol, Alkylthiol, Alkylsäure, Alkylpolyamin, Alkylpolyol und Alkylpolycarbonsäure.

32. Zusammensetzung nach Anspruch 30 oder 31, wobei die Alkoxysilane unter 3-Aminopropyltriethoxysilan, 3-Aminopropylmethyl-diethoxysilan, 3-[Bis(hydroxyethyl)aminopropyl]triethoxysilan ausgewählt sind.

33. Zusammensetzung nach einem der Ansprüche 28 bis 31, wobei der Vorläufer des Sol-Gel-Verfahrens das Tetraethylorthosilicat (TEOS) ist.

34. Zusammensetzung nach einem der Ansprüche 1 bis 24, wobei die Partikel, die in der erfindungsgemäßen Zusammensetzung enthalten sind, durch ein Verfahren erhältlich sind, mit dem man in einem wässrigen Medium die Kondensation von wenig oder nicht polymerisierten, in Wasser löslichen organischen Siliciumverbindungen, die unter den Organosilanen, die ein Siliciumatom enthalten, und unter den Organosiloxanen, die zwei oder drei Siliciumatome enthalten, ausgewählt sind, auf den Partikeln, welche überwiegend aus mindestens einem Metall gebildet sind und die dazu bestimmt sind, den Kern der Kern-Schale Partikel, die man zu erhalten wünscht, zu bilden bewirkt, wobei diese organischen Siliciumverbindungen ferner mindestens eine basische chemische Funktion und mindestens zwei hydrolysierbare Gruppen oder Hydroxygruppen pro Molekül, enthalten, wobei diese wenig oder nicht polymerisierten organischen Siliciumverbindungen, im Verhältnis von 1/1000 bis 99/100, vorzugsweise von 0,2/100 bis 70/100 durch ein Neutralisierungsmittel neutralisiert sind.

35. Zusammensetzung nach einem der Ansprüche 1 bis 24, wobei die umhüllten, eingekapselten Partikel, die die erfindungsgemäße Zusammensetzung mit einschließt, durch ein Verfahren hergestellt werden können, mit dem man in einem wässrigen Medium die Kondensation von wenig oder nicht polymerisierten, in Wasser löslichen organischen Siliciumverbindungen, die unter den Organosilanen, die ein Siliciumatom enthalten, und unter den Organosiloxanen, die zwei oder drei Siliciumatome enthalten, ausgewählt sind, auf den Partikeln, welche überwiegend aus mindestens einem Metall gebildet sind und die dazu bestimmt sind, den Kern der <umhüllten> Kern-Schale Partikel, die man zu erhalten wünscht, zu bilden bewirkt, wobei diese organischen Siliciumverbindungen ferner pro Molekül mindestens zwei Hydroxygruppen oder zwei hydrolysierbare funktionelle Gruppen und mindestens zwei nicht hydrolysierbare funktionelle Gruppen enthalten, wobei mindestens eine dieser nicht hydrolysierbaren funktionellen Gruppen eine funktionelle Gruppe ist, die eine kosmetische Wirkung hat und wobei mindestens eine andere dieser nicht hydrolysierbaren funktionellen Gruppen eine solubilisierende funktionelle Gruppe ist.

36. Zusammensetzung nach Anspruch 35, wobei die eine kosmetische Wirkung aufweisende(n) Gruppe(n) eine Gruppe mit färbender, vor UV-Strahlung schützender, antibakterieller oder fungizider Funktion oder eine mit reduzierender Funktion ist (sind).

37. Zusammensetzung nach einem der Ansprüche 1 bis 24, wobei die umhüllten, eingekapselten Partikel, die die erfindungsgemäße Zusammensetzung mit einschließt, durch ein Verfahren hergestellt werden können, mit dem man, in einem wässrigen Medium, die Kondensation von wenig oder nicht polymerisierten, in Wasser löslichen organischen Siliciumverbindungen, die unter den Organosilanen, die ein Siliciumatom enthalten, und unter den Organosiloxanen, die zwei oder drei Siliciumatome enthalten, ausgewählt sind, auf den Partikeln, welche überwiegend aus mindestens einem gediegenen Metall gebildet sind und die dazu bestimmt sind, den Kern der Kern-Schale Partikel, die man zu erhalten wünscht, zu bilden bewirkt, wobei diese organischen Siliciumverbindungen ferner mindestens eine nicht-basische solubilisierende chemische Funktion und mindestens zwei hydrolysierbare Gruppen pro Molekül enthalten.

38. Zusammensetzung nach einem der Ansprüche 1 bis 24, wobei die umhüllten, eingekapselten Partikel, die die erfindungsgemäße Zusammensetzung mit einschließt, durch ein Verfahren erhältlich sind, das die folgenden Schritte umfasst:
a) man stellt ein Gemisch her
(i) aus einer Dispersion von Partikeln, welche überwiegend aus mindestens einem gediegenen Metall gebildet werden und die dazu bestimmt sind, den Kern der Kern-Schale Partikel, die man zu erhalten wünscht, zu bilden, in einem flüssigem Medium, insbesondere einem wässrigen oder alkoholischen oder öligen Medium, gegebenenfalls in Gegenwart eines Dispergierungsmittels,
(ii) und einer Lösung eines vernetzenden hybriden organo-mineralischen Stoffes, wobei der genannte Stoff (vor Hydrolyse) mittels Sol-Gel-Verfahren erhalten wird, das von einer Vormischung ausgeht, die enthält:
(A) mindestens eine metallische oder Metall-organische Verbindung, und
(B) mindestens ein funktionalisiertes organisches Polymer oder dessen Vorläufer, oder mindestens ein funktionalisiertes Silicium-enthaltendes Polymer oder dessen Vorläufer, wobei letzteres anders als das in (A) ist,
b) dieses Gemisch wird mit Wasser in Kontakt gebracht, wenn die Dispersion der Partikel, die dazu bestimmt sind, den Kern zu bilden, eine Dispersion in einem flüssigen, alkoholischen oder öligen Medium ist.

39. Zusammensetzung nach einem der vorgenannten Ansprüche, wobei die äußere Oberfläche der Partikel in kovalenter Weise durch mindestens eine chemische Gruppe modifiziert ist, die geeignet ist, die Adsorption der Partikel auf Keratinsubstanzen wie Haaren, zu verbessern.

40. Zusammensetzung nach einem der vorgenannten Ansprüche, wobei die äußere Oberfläche der Partikel in kovalenter Weise durch mindestens eine chemische Gruppe modifiziert ist, die geeignet ist, mit Keratinsubstanzen wie Haaren, chemisch zu reagieren.

41. Zusammensetzung nach Anspruch 39, wobei die chemische Gruppe, die geeignet ist, die Adsorption der Partikel auf Keratinsubstanzen zu verbessern, unter den folgenden Gruppen ausgewählt ist:
- Carbonsäuren und deren Salzen,
- primären, sekundären, tertiären und quaternären Aminen,
- Phosphaten,
- Schwefeloxiden, wie Sulfonen, Sulfonsäuren, Sulfoxiden und Sulfaten,
- aromatischen Gruppen, wie Phenyl, Triazin, Thiophen und Imidazol.

42. Zusammensetzung nach Anspruch 40, wobei die chemische Gruppe, die geeignet ist, mit Keratinsubstanzen chemisch zu reagieren, unter den folgenden Gruppen ausgewählt ist:
- Epoxiden,
- Vinylen und aktivierten Vinylen: Acrylnitril, Acrylsäure- und Methacrylsäureestern, Crotonsäure und deren Estern, Zimtsäure und deren Estern, Styrol und dessen Derivaten, Butadien, Vinylether, Vinylketon, Estern der Maleinsäure, Maleimiden, Vinylsulfonen, ...
- Carbonsäuren und deren Derivaten: Anhydriden, Säurechloriden, Estern,
- Acetalen und Halbacetalen,
- Aminalen und Halbaminalen,
- Ketonen und Alpha-hydroxyketonen, Alpha-halogenketonen,
- Lactonen und Thiolactonen,
- Isocyanaten,
- Thiocyanaten,
- Iminen,
- Imiden, wie Succinimiden und Glutimiden,
- Pyridyldithio,
- N-Hydroxysuccinimidestern,
- Imidaten,
- Oxazinen und Oxazolinen,
- Oxazinium und Oxazolinium,
- Gruppen der Formel R₁X, wobei R₁ eine Alkylgruppe mit 1 bis 30 Kohlenstoffatomen, eine Arylgruppe mit 6 bis 30 Kohlenstoffatomen, eine Aralkylgruppe mit 7 bis 30 Kohlenstoffatomen (Alkylgruppe mit 1 bis 30 Kohlenstoffatomen) ist, und X eine austretende Gruppe bedeutet, wie I, Br, Cl, OSO₃R, wobei R H, eine Alkylgruppe mit 1 bis 30 Kohlenstoffatomen bedeutet, - SO₂R', wobei R' H, eine Alkylgruppe mit 1 bis 30 Kohlenstoffatomen bedeutet, eine Tosylgruppe, N(R")₃, wobei R" eine Alkylgruppe mit 1 bis 30 Kohlenstoffatomen bedeutet, OPO₃ (R"')₂, wobei R"' H oder eine Alkylgruppe mit 1 bis 30 Kohlenstoffatomen ist; bevorzugte Gruppen der Formel R₁X sind halogenierte Alkyle, Aryle oder Aralkyle;
- Gruppen der Formel R₂X, wobei R₂ einen Kohlenstoffring aus 3 bis 30 Kohlenstoffatomen oder einen 3 bis 20-gliedrigen ungesättigten Heterozyklus darstellt, der ein oder mehrere Heteroatome, welche aus N, S, O und P ausgewählt sind, enthält, und wobei X eine wie oben definierte austretende Gruppe ist; bevorzugte Gruppen der Formel R₂X sind halogenierte ungesättigte Ringe, wie Chlortriazin, Chlorpyrimidin, Chlorchinoxalin und Chlorbenzotriazol,
- Gruppen der Formel: R₃SO₂X, wobei R₃ die gleiche Bedeutung wie R₁ hat und X eine austretende Gruppe bedeutet, die die bereits oben angegebene Bedeutung hat,
- Lactonen,
- Thiolactonen,
- Siloxanen.

43. Zusammensetzung nach einem der Ansprüche 1 bis 38, wobei die Schale der Partikel aus einem reaktiven Metall-organischen Polymer gebildet ist, das in der Lage ist, zwischen den Partikeln kovalente Bindungen zu schaffen.

44. Zusammensetzung nach einem der vorhergehenden Ansprüche, wobei die Partikel in einer Konzentration im Bereich von 0,0001 bis 50 %, vorzugsweise im Bereich von 0,01 bis 5 % und noch bevorzugter im Bereich von 0,05 bis 2 %, bezogen auf die Gesamtmasse der Zusammensetzung, in der Zusammensetzung vorhanden sind.

45. Zusammensetzung nach einem der vorhergehenden Ansprüche, wobei das physiologisch akzeptable Medium mindestens ein Lösungsmittel enthält.

46. Zusammensetzung nach Anspruch 45, wobei das Lösungsmittel unter organischen Lösungsmitteln, Wasser und deren Gemischen ausgewählt ist.

47. Zusammensetzung nach Anspruch 46, wobei die organischen Lösungsmittel ausgewählt sind unter aliphatischen C₁₋₄-Alkoholen, wie Ethanol und Isopropanol, Polyolen, wie Glycerol oder Propylenglycol, aromatischen Alkoholen, wie Benzylalkohol, Alkanen, insbesondere C₅₋₁₀-Alkanen, Aceton, Methylethylketon, Methylacetat, Butylacetat, Alkylacetaten, Dimethoxyethan, Diethoxyethan und deren Gemischen.

48. Zusammensetzung nach einem der vorhergehenden Ansprüche, wobei sie ferner kosmetische Zusatzstoffe enthält, die unter Reduktionsmitteln, Oxidationsmitteln, Verdickungsmitteln, beruhigenden Stoffen, Schauminhibitoren, Direktfarbstoffen oder Oxidationsfarbstoffen, Parfums, Peptisierungsmitteln, Konservierungsstoffen, anionischen oder amphoteren grenzflächenaktiven Stoffen ausgewählt sind.

49. Zusammensetzung nach einem der vorhergehenden Ansprüche, wobei sie eine kosmetische Zusammensetzung zur Behandlung ist, insbesondere, um Glanz in Keratinsubstanzen zu bringen.

50. Zusammensetzung nach Anspruch 49, wobei sie eine Zusammensetzung zur Haarbehandlung ist, insbesondere, um Glanz in das Haar zu bringen.

51. Zusammensetzung nach Anspruch 50, wobei sie sich in Form einer Lotion, eines "Sprays", eines Schaums, eines Lacks, einer Kurspülung oder eines Shampoos vorliegt.

52. Zusammensetzung nach einem der vorhergehenden Ansprüche, wobei sie in einer Aerosolvorrichtung konfektioniert ist.

53. Kosmetisches Verfahren zur Behandlung von Keratinsubstanzen wie Haaren, insbesondere, um Glanz in Keratinsubstanzen, wie Haare, zu bringen, das das Aufbringen einer Zusammensetzung nach einem der Ansprüche 1 bis 52 auf Keratinsubstanzen wie Haaren, umfasst.

54. Verwendung der Zusammensetzung nach einem der Ansprüche 1 bis 52, um Glanz in Keratinsubstanzen, wie Haare, zu bringen.

55. Verwendung der in einem der Ansprüche 1 bis 52 beschriebenen Partikel in einer kosmetischen Zusammensetzung, um Glanz in Keratinsubstanzen, wie Haare, zu bringen.
